(19) Europäisches Patentamt
European Patent Office
Office européen des brevets

(11) Veröffentlichungsnummer: **0 358 608 A2**

(12) **EUROPÄISCHE PATENTANMELDUNG**

(21) Anmeldenummer: **89810649.7**

(51) Int. Cl.[5]: **C 07 H 19/01**
A 01 N 43/90

(22) Anmeldetag: **01.09.89**

(30) Priorität: **09.09.88 CH 3375/88**

(43) Veröffentlichungstag der Anmeldung:
**14.03.90 Patentblatt 90/11**

(84) Benannte Vertragsstaaten:
**AT BE CH DE ES FR GB GR IT LI LU NL SE**

(71) Anmelder: **Gesellschaft für Biotechnologische Forschung mbH (GBF)**
**Mascheroder Weg 1**
**D-3300 Braunschweig-Stöckheim (DE)**

**CIBA-GEIGY AG**
**Klybeckstrasse 141**
**CH-4002 Basel (CH)**

(72) Erfinder: **Böhlendorf, Bettina**
**Zimmerstrasse 2**
**D-3300 Braunschweig (DE)**

**Bedorf, Norbert, Dr.**
**Krukenbergstrasse 4**
**D-3308 Königslutter (DE)**

**Höfle, Gerhard, Prof. Dr.**
**Alter Weg 12 a**
**D-3300 Braunschweig (DE)**

**Schummer, Dietmar**
**Eichtalstrasse 1**
**D-3300 Braunschweig (DE)**

**Sutter, Marius, Dr.**
**Klingental 5**
**CH-4058 Basel (CH)**

Die Bezeichnung der Erfindung wurde geändert (Richtlinien für die Prüfung im EPA, A-III, 7.3).

(54) **Mikrobizide makrozyklische Laktonderivate.**

(57) Neue macrocyclische Verbindungen der Formel I

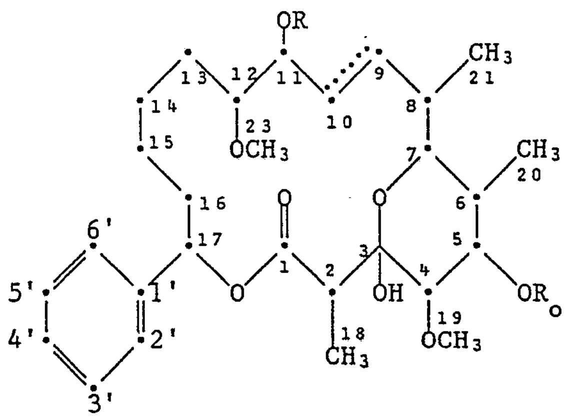

(I)

worin die gepunktete Linie in 9,10-Stellung wahlweise eine gesättigte Bindung oder eine Doppelbindung bedeutet, und R Wasserstoff, Methyl oder bestimmte Acylreste bedeutet, und $R_0$ Wasserstoff, Halogen, Azido, Thiol, Hydroxy oder -OY bedeutet, worin Y unter anderen Resten einen Ether-Rest, eine Silylgruppe, eine Sulfonylgruppe, einen Zuckerrest oder einen (Thio)Acylrest darstellt, wobei R Methyl bedeutet, wenn in 9,10-Stellung eine Doppelbindung vorliegt, besitzen mikrobizide Eigenschaften zur Bekämpfung pflanzenpathogener Mikroorganismen. Sie können in üblicher Formulierung zur Bekämpfung von Pflanzenkrankheiten eingesetzt werden.

EP 0 358 608 A2

**Beschreibung**

## Mikrobizide

Die vorliegende Erfindung betrifft eine macrocyclische Verbindung der Formel I, ein Verfahren zu ihrer Herstellung und ihre Verwendung zur Bekämpfung von Pflanzenkrankheiten sowie pflanzenmikrobizide Mittel, die diese Verbindung als Wirkstoff enthalten.

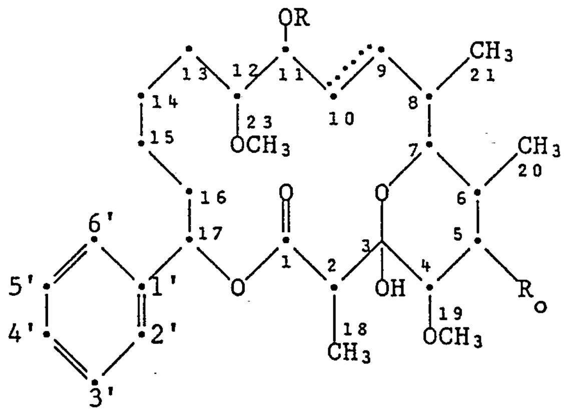

(I)

In dieser Formel bedeutet die gepunktete Linie in 9,10-Stellung wahlweise eine gesättigte Bindung oder eine Doppelbindung, während
R Wasserstoff, $CH_3$ oder -COA ist, worin
A Wasserstoff, $C_3$-$C_6$Cycloalkyl oder unsubstituiertes oder durch Halogen oder $C_1$-$C_3$Alkoxy substituiertes $C_1$-$C_6$Alkyl darstellt, und
$R_0$ Wasserstoff, Halogen, $-N_3$, -SH, -OH oder -OY bedeutet, worin
Y wahlweise

a) eine $C_3$-$C_6$Alkenyl- oder Alkinylgruppe, eine unsubstituierte oder substituierte $C_1$-$C_6$Alkylgruppe, eine Alkoxyalkoxyalkylgruppe mit bis zu 10 C-Atomen, eine $C_3$-$C_6$Cycloalkylgruppe oder eine unsubstituierte oder substituierte Phenylgruppe bedeutet, oder

b) eine Silylgruppe $-SiR_1R_2R_3$ darstellt, in der $R_1$-$R_3$ unabhängig Alkyl, Phenyl, Benzyl oder $C_3$-$C_6$Cycloalkyl bedeuten, oder

c) $-SO_2Z$ darstellt, worin Z die Gruppe -OM bedeutet, in der M Wasserstoff oder das Moläquivalent eines Metalles ist, oder worin Z $C_1$-$C_6$Alkyl oder unsubstituiertes oder substituiertes Aryl bedeutet; oder

d) eine Gruppe

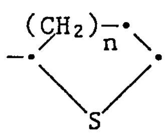

mit n = 1 oder 2 oder eine unsubstituierte oder nach Art einer Furanose oder Pyranose ganz oder teilweise durch -OH substituierte Gruppe

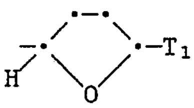 oder 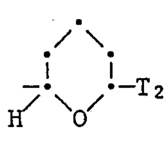

in der $T_1$ Wasserstoff, -OH, $-CH_2OH$ oder $-CHOH-CH_2OH$ und $T_2$ Wasserstoff, -OH oder $-CH_2OH$ sind, darstellt, oder

e) eine (Thio)Acylgruppe -CO-B oder -CS-B bedeutet, worin
B Wasserstoff, unsubstituiertes oder substituiertes $C_1$-$C_6$Alkyl, oder aber $C_2$-$C_6$Alkenyl, $C_2$-$C_6$Alkinyl, $C_3$-$C_6$Cycloalkyl, unsubstituiertes oder substituiertes Phenyl oder eine der Gruppen $-N(R_4)(R_5)$, $-OR_6$ oder $-SR_6$ darstellt,
$R_4$ und $R_5$ unabhängig voneinander Wasserstoff, $C_1$-$C_6$Alkyl, $C_1$-$C_6$Haloalkyl, $C_3$-$C_6$Alkenyl, $C_3$-$C_6$Alkinyl, $C_3$-$C_6$Cycloalkyl oder Phenyl bedeuten, und
$R_6$ $C_1$-$C_6$Alkyl, $C_3$-$C_6$Cycloalkyl oder Phenyl ist, mit der Massgabe, dass R Methyl bedeutet, wenn in 9,10-Stellung eine Doppelbindung vorliegt, und dass R die Gruppe -COA darstellt, wenn $R_0$ eine OH-Gruppe ist.

Bedeutet Y eine substituierte $C_1$-$C_6$Alkylgruppe, so können Halogen, Hydroxy, $C_1$-$C_6$Alkoxy, $C_1$-$C_6$Alkylthio, Carboxy (= COOH), Amino, $C_3$-$C_6$Cycloalkyl, Phenyl oder substituiertes Phenyl, Phenoxy oder substituiertes Phenoxy die Substituenten sein. Im allgemeinen enthält eine Alkylgruppe jedoch höchstens ein oder zwei aromatische Ringe.

Eine substituierte Phenyl- oder Phenoxy-Gruppe kann ein- oder mehrfach durch gleiche oder verschiedene Substituenten wie $C_1$-$C_4$Alkyl, Halogen, Hydroxy, $C_1$-$C_4$Alkoxy, $CF_3$ oder $NO_2$ substituiert sein, wobei es für den Fachmann selbstverständlich ist, dass raumerfüllende Substituenten wie tert. Butyl höchstens dreimal oder zum Zerfall neigende Substituenten wie $NO_2$ höchstens zweimal im Phenylring auftreten, wohingegen andere Substituenten wie Fluor oder Methyl bis zu fünffach vertreten sein können.

Unter Halogen werden Fluor, Chlor, Brom oder Jod verstanden.

Aryl bedeutet Phenyl, Naphthyl oder Diphenyl. Unter $C_3$-$C_6$Cycloalkyl werden Cyclopropyl, Cyclobutyl, Cyclopentyl und Cyclohexyl verstanden.

Unter Alkyl werden je nach Kettenlänge Methyl, Ethyl, Propyl, Butyl, Amyl, Hexyl, Heptyl, Octyl, Nonyl, Decyl sowie ihre Isomeren, z.B. Isopropyl, Isobutyl, sek. Butyl, tert. Butyl, Neopentyl usw. verstanden.

Ein durch Halogen substituierter Alkylrest steht für einen einfach bis perhalogenierten Alkylsubstituenten wie $CHCl_2$, $CH_2Cl$, $CCl_3$, $CF_3$ $CH_2F$, $CH_2Br$, $CH_2CH_2Cl$, $CHF$-$CH_3$, $CHBr_2$ usw.

Beispiele für Alkylreste, die durch Alkoxy auch im Sinne einer Alkoxyalkoxy-Substitution ein- oder mehrfach substituiert sind, seien $-CH_2OCH_3$, $-CH_2CH_2OCH_3$, $-CH_2CH(CH_3)OCH_3$, $-CH_2OC_2H_5$, $-CH_2OC_3H_7$-i, $-CH_2CH_2CH_2OCH_3$, $-CH_2OCH_2OCH_3$, $-CH_2CH_2OCH_2OCH_3$, $-CH_2OCH_2CH_2OCH_3$, $-CH_2OCH_2OO_2H_5$, $-C(CH_3)_2$-$CH_2OCH_3$, $-CH(CH_3)OCH_2OC_3H_7$-i, $-CH(OCH_3)$-$CH_2OCH_3$ und andere verzweigte oder unverzweigte Reste.

Alkenyl steht für einen aliphatischen Kohlenwasserstoffrest mit einer Doppelbindung, z.B. für Vinyl, Propen(1)-yl, Allyl, Buten(1)-yl, Buten(2)-yl, Hexen(2)-yl usw.

Alkinyl steht für einen aliphatischen Kohlenwasserstoffrest mit einer Dreifachbindung, z.B. für Ethinyl, Propin(1)-yl, Propargyl, Butin(1)-yl usw.

Die für Y unter d) genannten Tetrahydrofurane und Tetrahydropyrane sowie ihre Thio-Analogen schliessen Monosaccharide ein, beispielsweise Glucose, Fructose, Altrose, Mannose, Sorbose, Gulose, Idose, Allose, Galactose, Ribose, Arabinose, Xylose, Lyxose, Erythrose, Threose, Rhamnose, Altrose, Talose.

Die Verknüpfung eines Saccharids in 5-Position der Verbindungen der Formel I kann als α-Anomeres oder β-Anomeres erfolgen. Die vorliegende Erfindung bezieht sich auf beide Bindungsarten.

Sofern Y eine wie unter e) definierte Gruppe $-CO-N(R_4)(R_5)$ oder $-CO-OR_6$ bzw. $-CO-SR_6$ bedeutet, stellen die Verbindungen der Formel I Carbaminsäureester oder Kohlensäureester bzw. Thiokohlensäureester dar.

Als Metalle für den Substituenten M kommen vor allem Alkali- und Erdalkali-Metalle wie Li, Na, K, Ca, Mg, sowie Al, Co, Ni, Zn, Cu in Frage.

Die Formel I umfasst sämtliche möglichen Stereoisomeren.

Die Verbindungen der Formel I leiten sich von der "Soraphen A" und "Soraphen B" genannten Grundstruktur neuer macrocylischer Verbindungen der nachstehenden Formel ab

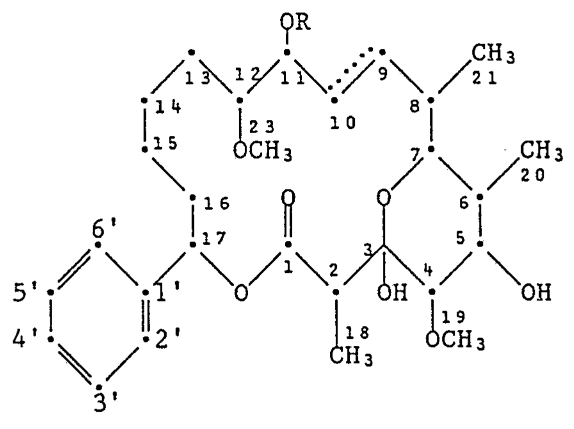

In dieser Formel bedeutet R Methyl, wenn in 9,10-Stellung eine Doppelbindung steht (= Soraphen A) oder R bedeutet Wasserstoff, wenn in 9,10-Stellung eine Einfachbindung steht (= Soraphen B). Weiterhin umfasst die Formel I Derivate des 9,10-Dihydro-Soraphen A, dass durch Hydrierung von Soraphen A gewonnen wird.

Aufgrund der physikochemischen Daten wird angenommen, dass diesen beiden Präparaten folgende Konfiguration zukommt:

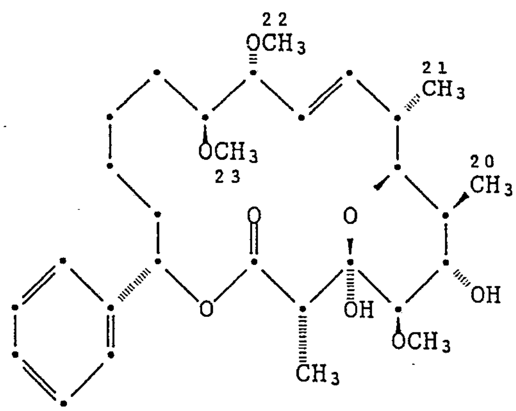

Soraphen A

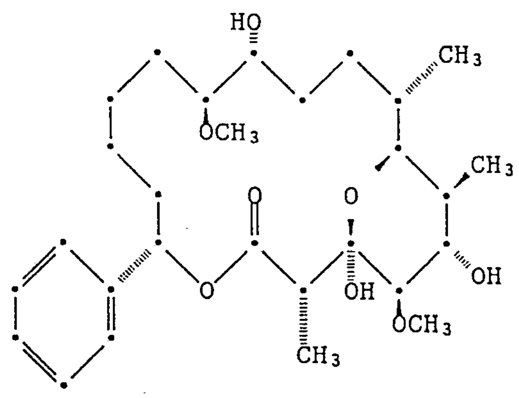

Soraphen B

Sorphen A und B werden durch mikrobiologische Kultivierung eines Sorangium (Polyangium) cellulosum-Stammes "So ce 26" gewonnen. Dieser Stamm wurde am 5. März 1987 bei der "National Collection of Industrial and Marine Bacteria (NCIB)", Torry Research Station, Aberdeen, Grossbritannien, unter der Nummer NCIB 12 411 gemäss Budapester Vertrag hinterlegt. Sorangium cellulosum gehört zur Ordnung der Myxobacterales, Unterordnung Sorangineae, Familie Polyangiaceae.

"So ce 26" selbst oder Mutanten oder Rekombinanten sind Gegenstand der europäischen Patentanmeldung EP-A-0282 455. Der Stamm lässt sich nach üblichen biologischen Methoden, z.B. in Schüttelkulturen oder in Fermentoren mit Nährmedien bei 10-35° C und einem pH-Wert von 6-8, kultivieren. Der Prozess verläuft aerob. Die Bedingungen zur Kultivierung des Mikroorganismus werden per Referenz zur EP-A-0282 455 in die vorliegende Beschreibung eingeführt.

In Verbindungen der Formel I vorliegender Erfindung bzw. bei entsprechenden Zwischenprodukten ist die Reaktionsfähigkeit einer Hydroxylgruppe in 5-Stellung verschieden von der einer Hydroxylgruppe in 11-Stellung des Moleküls. Damit ist die Zugänglichkeit von z.B. in diesen Positionen unterschiedlich acylierten oder partiell acylierten Verbindungen der Formel I aus "Soraphen A", "9,10-Dihydro-Soraphen A" bzw. "Soraphen B" gewährleistet. Andernfalls lassen sich Acylierungsprodukte z.B. chromatographisch voneinander trennen.

Eine wichtige Untergruppe von Verbindungen der Formel I sind solche, bei denen
R $CH_3$ und
$R_0$ Halogen, $-N_3$, -SH, -OH oder -OY bedeuten, worin Y die unter a), b), c), d) und e) genannten Bedeutungen besitzt. Diese Gruppe soll hier und im folgenden Untergruppe IA genannt werden.

Unter den Verbindungen der Untergruppe IA stellen solche eine besondere Gruppe dar, bei denen
Y die unter a) genannte Bedeutung hat (= Untergruppe IB).

Eine andere wichtige Untergruppe sind jene der Formel IA, bei denen Y die unter b) genannte Bedeutung hat (= Untergruppe IC).

Eine weitere wichtige Untergruppe sind jene der Formel IA, bei denen Y die unter d) genannte Bedeutung hat (= Untergruppe ID), insbesondere jene, bei denen Y einen durch Hydroxy und $-CH_2OH$ substituierten Tetrahydrofuranyl- oder Tetrahydropyranyl-Ring darstellt (= Untergruppe IDD).

Eine weitere wichtige Untergruppe sind jene der Formel IA, bei denen Y die unter e) genannte Acylierung aufweist (= Untergruppe IE).

Eine andere Reihe wichtiger Verbindungen der Formel I sind solche, bei denen
R Wasserstoff oder die Gruppe -COA darstellt, worin
A Wasserstoff, $C_3$-$C_6$Cycloalkyl, unsubstituiertes oder durch Halogen oder $C_1$-$C_3$Alkoxy substituiertes $C_1$-$C_6$Alky bedeutet, während

$R_0$ Wasserstoff, $-N_3$, Halogen, OH oder -SH ist. Diese Gruppe soll hier und im folgenden Untergruppe IIa genannt werden.

Unter diesen stellen solche eine wichtige Gruppe dar, bei denen A eine unsubstituierte oder durch Halogen oder $C_1$-$C_3$Alkoxy substituierte $C_1$-$C_4$Alkylgruppe bedeutet (= Untergruppe IIB).

Zu den als Mikrobizide bevorzugten Einzelverbindungen gehören:

Soraphen-A-5-propionat (Verb. Nr. 6)
Soraphen-A-5-butyrat (Verb. Nr. 7)
Soraphen-A-5-isobutyrat (Verb. Nr. 8)
Soraphen-A-5-acetat (Verb. Nr. 4)
Soraphen-A-5-formiat (Verb. Nr. 1)
Soraphen-A-5-trichloracetat (Verb. Nr. 25)
Soraphen-A-5-succinat (Verb. Nr. 55)
Soraphen-A-5-tiglat (Verb. Nr. 49)
Soraphen-A-5-methoxyacetat (Verb. Nr. 47)
Soraphen-A-5-β-D-glucopyranosid (Verb. Nr. 136) und
Soraphen-A-5-(5R)-β-D-glucopyranosid (Verb. Nr. 136)
Soraphen-A-5-pivalat (Verb. Nr. 171)
9,10-Dihydro-Soraphen-A-5-methoxyacetat (Verb. Nr. 113)
9,10-Dihydro-Soraphen-A-5-formiat (Verb. Nr. 18)
9,10-Dihydro-Soraphen-A-5-trichloracetat (Verb.Nr. 175)
9,10-Dihydro-Soraphen-A-5-β-D-glucopyranosid (Verb. Nr. 124)
und
9,10-Dihydro-Soraphen-A-5-(5R)-β-D-glucopyranosid (Verb. Nr. 124)
Soraphen-B-5,11-diacetat (Verb. Nr. 11)
Soraphen-B-5-formiat (Verb. Nr. 2)
Soraphen-B-5,11-diformiat (Verb. Nr. 3)
Soraphen-B-5-trichloracetat (Verb. Nr. 176)
Soraphen-A-5-aminoacetat (Verb. Nr. 53)
Soraphen-B-5-aminoacetat (Verb. Nr. 178)
9,10-Dihydro-Soraphen-A-5-aminoacetat (Verb. Nr. 177).

Verbindungen der Formel I lassen sich in der 5- bzw. 11-Position, ausgehend vom "Soraphen A" oder "Soraphen B", nach Methoden derivatisieren, die gleichfalls Gegenstand vorliegender Erfindung sind.

Das Verfahren zur Herstellung von Verbindungen der Formel I ist gekennzeichnet durch Verätherung, Silylierung oder Acylierung der 5-Hydroxygruppe im "Soraphen A", im "9,10-Dihydro-Soraphen A" oder im "Soraphen B" und/oder durch Acylierung der 11-Hydroxygruppe im "Soraphen B".

Diese drei Präparate "Soraphen A", "9,10-Dihydro-Soraphen A" und "Soraphen B" sollen als Ausgangsprodukte für die Derivatisierungen hier und im folgenden vereinfachend "Soraphen" genannt werden.

Durch übliche Acylierung einer OH-Gruppe mit der entsprechenden (Thio)Carbonsäure bzw. mit einem entsprechenden Acylhalogenid, Acylanhydrid oder Ester oder mit der entsprechenden Sulfonsäure oder ihrem Acylhalogenid oder durch Reaktion einer OH-Gruppe mit dem entsprechend substituierten Silan-Derivat der Formel

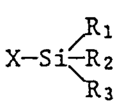

werden alle jene Derivate gewonnen, bei denen R die Gruppe -COA bedeutet bzw. $R_0$ die Gruppe -OY darstellt, in der Y eine der unter b), c) oder e) genannten Bedeutungen hat, wobei der Begriff Acylhalogenid, der auch Sulfonsäurehalogenid einschliesst, für Acylchlorid oder Acylbromid steht und wobei X eine Silylabgangsgruppe bedeutet. Zu den Silylabgangsgruppen X zählen beispielsweise Bromid, Chlorid, Trifluormethansulfonat. Diese Aufzählung stellt keine Limitierung dar. Der Fachmann kennt weitere typische Silylabgangsgruppen. Als geeignete Silylgruppen $-SiR_1R_2R_3$ kommen beispielsweise Trimethylsilyl, Diphenyl-tert.butylsilyl, bis(Isopropyl)-methylsilyl, Triphenylsilyl usw. und insbesondere tert.Butyl-di-methylsilyl in Frage.

O-Acylierungen und O-Silylierungen werden in wasserfreiem Milieu, vorzugsweise in inerten Lösungsmitteln und besonders bevorzugt in aprotischen Lösungsmitteln durchgeführt. Die Reaktion läuft vorteilhaft im Temperaturbereich von 0°C bis 80°C, bevorzugt bei 10°C bis 50°C ab.

Vorzugsweise wird eine organische Base zugegeben. Genannt seien beispielsweise tertiäre Amine wie Triethylamin, Triethylendiamin, Triazol und bevorzugt Pyridin, Imidazol oder 1,8-Diazabicyclo[5.4.0]-undec-7-en (DBU).

Geeignete Lösungsmittel sind z.B: Ether und etherartige Verbindungen wie Dialkylether (Diethylether, Diisopropylether, tert.-Butylmethylether, Dimethoxiethan, Dioxan, Tetrahydrofuran, Anisol, usw.); halogenierte Kohlenwasserstoffe wie Chlorbenzol, Methylenchlorid, Ethylenchlorid, Chloroform, Tetrachlorkohlenstoff, Tetrachlorethylen, usw.; oder Sulfoxide wie Dimethylsulfoxid, wobei auch aromatische oder aliphatische Kohlenwasserstoffe, wie Benzol, Toluol, Xylole, Petrolether, Ligroin, Cyclohexan, usw. anwesend sein können. In manchen Fällen kann es von Vorteil sein, wenn die Reaktionen unter Schutzgasatmosphäre (z.B. Argon,

Helium, Stickstoff, etc.) und/oder in absoluten Lösungsmitteln durchgeführt werden.

Setzt man zur Acylierung eine freie Carbonsäure oder Sulfonsäure als Reaktand ein, so wird diese Reaktion zweckmässigerweise in Gegenwart wasserabspaltender Reagenzien durchgeführt. Man arbeitet beispielsweise in Anwesenheit von Dicyclohexylcarbodiimid und Pyridin oder in Gegenwart von Azodicarbonsäuredialkylester und Triphenylphosphin.

Werden Säurehalogenide oder Säureanhydride zur Acylierung eingesetzt, so erweist sich der Zusatz eines Neutralisationsmittels als vorteilhaft. Tertiäre Amine wie Trialkylamine, Pyridin oder Pyridinbasen wie 4-Dimethylaminopyridin sind zweckmässige Reagenzien, die zum Teil auch als Lösungsmittel dienen können.

Sofern in der 5-Position eine Sulfonsäuregruppe oder ihr Salz $-SO_3M$ eingeführt werden soll, lässt man Soraphen mit dem $[SO_3$/Triethylamin]-Komplex in Gegenwart einer Base wie Alkalihydroxyd, Alkalicarbonat oder NaH bei 0° bis 150° C reagieren, wobei man vorteilhaft ein Lösungsmittel wie Halogenkohlenwasserstoff, z.B. Chloroform, zusetzt. [W.B. Hardy et al. J. Am. Chem. Soc. 74, 5212 (1952).

Die Herstellung von Verbindungen der Formel I, die in der 5-Position an das Sauerstoffatom einen Zuckerrest gebunden haben, wird nach einem der in der Zuckerchemie verwendeten Verknüpfungsmethoden, z.B nach der Koenigs-Knorr-Synthese, dem Ag-Triflat-Prozess, dem Orthoester-Verfahren, der Phenylthio-Synthese oder der 2-Pyridylthio-Synthese durchgeführt.

A) Nach der Koenigs-Knorr-Synthese oder dem Silber-Triflat-Prozess lässt sich die 5-Hydroxy-Gruppe der Verbindung der Formel I ($R_0 = OH$) in Gegenwart eines Silbersalzes oder Quecksilber-Salzes als Kondensationsmittel mit dem einzuführenden Zuckerrest, worin sämtliche OH-Gruppen durch z.B. Acetylierung geschützt sind mit Ausnahme der durch Chlor oder Brom substituierten 1-OH-Gruppe des Zuckerrestes, im Temperaturbereich von -30° C bis +60° C, bevorzugt -5° C bis +30° C, unter Licht-Ausschluss gewinnen.

Der geschützte 1-Chlor- oder 1-Brom-Zucker wird in mindestens äquimolarer Menge zum Soraphen gegeben, vorzugsweise jedoch im 1,5- bis 3-fachen Ueberschuss.

Als Silbersalz lässt sich frisch gefälltes $Ag_2O$ verwenden, $Ag/Ag_2O$ auf $Al_2O_3$ abgeschieden, vorzugsweise jedoch $Ag_2CO_3$ oder $CF_3$-COOAg. Besonders bevorzugt ist Silber-Trifluormethansulfonat (=Ag-Triflat = $CF_3-SO_3Ag$), in dessen Gegenwart die Glykosidierung bereits bei Minustemperaturen schnell abläuft. Zum Aktivieren der 5-OH-Gruppe und zum Neutralisieren der gegebenenfalls entstehenden $CF_3-SO_3H$ bzw. $CF_3$-COOH setzt man zweckmässig ein tert.-Amin (Triethylamin, Diisopropylethylamin, Diazabicycloundecan u.a.) der Reaktionslösung zu.

Anstatt des Silbersalzes lässt sich auch Hg-Cyanid oder eine Kombination von HgO mit wahlweise Hg-Chlorid oder Hg-Bromid verwenden (Helferich-Synthese).

Nach einer weiteren Variante lässt sich die Reaktivität in der 1′-Stellung des glykosidisch zu verknüpfenden Zuckers, dessen weitere OH-Gruppen geschützt sein müssen, durch anfängliche Umwandlung in das 1′-Phenylthio-Derivat und anschliessende Reaktion mit DAST (= Diethylaminoschwefeltrifluorid) in absolut trockenem Dichlormethan (Molekularsieb) bei +5° C bis -30° C zum 1′-Fluorderivat erhöhen. Reaktiver als das entsprechende, bei der Koenigs-Knorr-Synthese eingesetzte 1′-Chlor- oder 1′-Brom-Derivat lässt sich das so gewonnene 1′-Fluor-Derivat des Zucker-Reaktanden mit der 5-Hydroxygruppe des Soraphen in Gegenwart von $SnCl_2$ und $AgClO_4$ in einem trockenen aprotischen Lösungsmittel wie Diethylether unter Schutzgas wie Argon bei +5° C bis -30° C verknüpfen. (J. Am. Soc. 1984, 106, 4189-4192).

B) Man kann auch das geschützte, in 1′-Stellung zu aktivierende Monosaccharid mit 2,2′-Dithiopyridin in trockenem Dichlormethan bei -10° C bis +10° C und unter Schutzgas-Atmosphäre (z.B. Argon) in das 1′-S-(2-Pyridyl)-Monosaccharid überführen, das mit der freien 5-OH-Gruppe des Soraphen in Gegenwart von $Pb(ClO_4)_2$ oder $AgClO_4$ als Kondensationsmittel bei Raumtemperatur in Tetrahydrofuran (THF) als Lösungsmittel leicht unter Bildung der glykosidischen Bindung reagiert. (J. Org. Chem. 1983, 48, 3489-3493).

C) Glykosidische Verknüpfungen lassen sich auch in Gegenwart von Lewis-Säuren erzielen, wie $AlCl_3$, $AlBr_3$, $SnCl_4$, $ZnCl_2$, $BF_3$ (sowie vor allem das Etherat), wozu insbesondere acetylierte Zucker sehr geeignet sind. (Chimia 21, 1967, S. 537-538).

D) Nach der sogenannten Orthoester-Methode lassen sich glykosidische Bindungen auch durch Reaktion von Soraphen mit dem zu verknüpfenden geschützten Zucker in Gegenwart des Orthoesters eines niederen Alkohols erzielen, dessen eine alkoholische Komponente der Zucker-Reaktand ist.

Das Verfahren zur Herstellung von Soraphen-5-Glykosiden der Formel I, ist im engeren Sinne gekennzeichnet durch Reaktion von Soraphen

a) in Gegenwart eines Silbersalzes oder Quecksilber-Salzes als Kondensationsmittel mit dem einzuführenden Zuckerrest, dessen sämtliche OH-Gruppen geschützt sind, mit Ausnahme der in 1-Stellung durch Chlor oder Brom substituierten anomeren 1-OH-Gruppe unter Licht-Ausschluss im Temperaturbereich von -30° C bis +60° C, bevorzugt -5° C bis +30° C; oder

b) in Gegenwart von $SnCl_2$ und $AgClO_4$ als Kondensationsmittel mit dem einzuführenden Zuckerrest, dessen sämtliche OH-Gruppen geschützt sind, mit Ausnahme der in 1-Stellung durch Fluor substituierten anomeren 1-OH-Gruppe unter Lichtausschluss bei +5° C bis -30° C; und anschliessende milde Verseifung der Hydroxy-Schutzgruppen am Zuckerrest.

Die Schutzgruppen lassen sich in der Regel durch milde Verseifung mit z.B. $NH_3$/Methanol abspalten. Als Lösungsmittel kommen bei diesem Teilschritt insbesondere wasserfreie aprotische Vertreter wie Dichlormethan, Acetonitril, Benzol, Toluol, Nitromethan, Dioxan, THF, Ethylenglykoldimethylether in Frage; Diethylether ist besonders geeignet.

Verbindungen der Formel I, in denen $R_0$ eine Carbamoyloxygruppe -O-CO-N($R_4$)($R_5$) oder eine Thiocarbamoylgruppe -O-CS-N($R_4$)($R_5$) bedeutet, lassen sich durch Carbamoylierung von Soraphen mit

a) einem Isocyanat $R_5$-NCO oder einem Isothiocyanat $R_5$-NCS in den Fällen gewinnen, in denen $R_4$ für Wasserstoff steht, oder mit

b) einem (Thio)carbaminsäurehalogenid der Formel

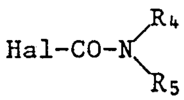 bzw. 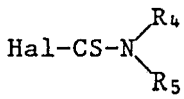

gewinnen,

worin $R_4$ und $R_5$ unabhängig voneinander eine der angegebenen Bedeutungen haben und Hal für Halogen, vorzugsweise Chlor oder Brom, steht.

Nach einer weiteren Variante c) lässt sich die 5-Hydroxygruppe des Soraphen mit Phosgen bzw. Thiophosgen in Gegenwart eines säurebindenden Mittels wie Pyridin in einen Chlorkohlensäureester bzw. Chlorthiokohlensäureester

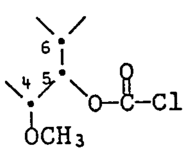 bzw. 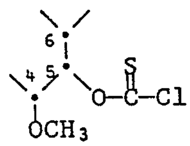

überführen, aus dem man durch Reaktion mit einem primären oder sekundären Amin

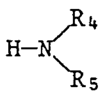

das gewünschte Carbamat erhält.

Geeignete Iso(thio)cyanate sind beispielsweise Trichloracetylisocyanat zur Einführung der unsubstituierten Carbamoylgruppe (-CO-$NH_2$), gefolgt von milder basischer Abspaltung des $CCl_3$-CO-Restes, dann ferner Methylisocyanat, Trifluormethylisocyanat, tert. Butylisocyanat, Chloräthylisocyanat, Allylisocyanat, Phenylisocyanat, Cyclopropylisocyanat, Cyclohexylisocyanat, Phenylisothiocyanat, Methylisothiocyanat, Isopropylisothiocyanat.

Geeignete Beispiele von Carbamoylhalogeniden sind Dimethylcarbaminsäurechlorid, Diäthylcarbaminsäurechlorid, Diisopropylcarbaminsäurechlorid, Diphenylcarbaminsäurechlorid und Di-sek.-butylcarbaminsäurechlorid.

Die Carbamoylierungen mit Iso(thio)cyanaten und Carbamoylhalogeniden werden in inerten hydroxylgruppenfreien Lösungsmitteln vorzugsweise in Anwesenheit eines tertiären Amins wie z.B. Pyridin, 4-Dimethylaminopyridin, Lutidin, Collidin, Trialkylamin, N-Dialkylanilin, oder einer bicyclischen, nicht nucleophilen Base wie z.B. 1,4-Diazabicyclo[2.2.2]octan (DABCO), 1,5-Diazabicyclo[4.3.0]-non-5-en (DBN) oder 1,8-Diazabicyclo[5.4.0]undec-7-en (1,5-5) (DBU) durchgeführt.

Die Reaktion wird im allgemeinen bei Temperaturen von -10°C bis +150°C bei Einsatz von Iso(thio)cyanaten, vorzugsweise 10°C bis 120°C, durchgeführt, bei Einsatz von Carbamoylhalogeniden vorzugsweise bei +50°C bis 120°C.

Sofern die Reaktion nicht im Ueberschuss einer der oben genannten tertiären Amine durchgeführt wird, empfehlen sih beispielsweise folgende allgemeine Lösungsmittel oder Lösungsmittelgemische:

Aliphatische und aromatische Kohlenwasserstoffe wie Benzol, Toluol, Xylole, Petrolether, Hexan; halogenierte Kohlenwasserstoffe wie Chlorbenzol, Methylenchlorid, Ethylenchlorid, Chloroform, Tetrachlorkohlen stoff, Tetrachlorethylen; Ether und etherartige Verbindungen wie Dialkylether (Diethylether, Diisopropylether, tert.-Butylmethylether usw.), Anisol, Dioxan, Tetrahydrofuran; Nitrile wie Acetonitril, Propionitril; Ester wie Ethylacetat, Propylacetat oder Butylacetat; Ketone wie Aceton, Diethylketon, Methylethylketon; Dimethylsulfoxid (DMSO); Dimethylformamid (DMF) und andere. Diese allgemeinen Lösungsmittel gelten auch für die folgenden Reaktionen, sofern darauf Bezug genommen wird.

Sofern die 5-OH-Gruppe im Soraphen in den Substituenten $R_o$ = Halogen oder Azido überführt werden soll, so geschieht dies zweckmässig über die Stufe der weiter oben beschriebenen 5-Sulfonsäureester. Diese lassen sich im Temperaturbereich von 0° bis 170°C in Lösungsmitteln wie Aceton, Niederalkanol, Ethylenglykol oder eines der oben genannten allgemeinen Lösungsmittel mit Alkali- oder Erdalkali-Halogeniden bzw. -Aziden, z.B. LiBr, KJ, $MgBr_2$, KF, $NaN_3$, in die entsprechende 5-Halogenverbindung bzw. 5-Azidoverbindung überführen.

Thiol-Derivate ($R_o$ = -SH) lassen sich durch Reaktion eines entsprechenden Sulfonsäureesters mit einem Thioester (z.B. Kaliumthioacetat) in basischem Milieu und einem geeigneten Lösungsmittel wie Aceton oder eines der oben genannten allgemeinen Lösungsmittel gewinnen. Der gebildete Thiocarbonsäureester kann unter basischen Bedingungen (z.B. verd. alkoholische oder wässrige Lauge) bei Raumtemperatur oder unter

sauren Bedingungen (z.B. methanolische Salzsäure) bei erhöhter Temperatur gespalten werden.

5-Desoxyverbindungen ($R_o$=H) können ausgehend von den entsprechenden Thiolen durch Reaktion mit Raney-Nickel in geeigneten Lösungsmitteln wie Alkoholen, 1,4-Dioxan etc. bei Temperaturen von 10° bis 100°C erhalten werden. Ein anderer Zugang besteht in der Umsetzung von Soraphen mit Schwefelkohlenstoff ($CS_2$) in geeigneten Lösungsmitteln wie z.B. Aceton, Aether, etc. unter basischen Bedingungen (z.B. KOH, NaH, etc.) bei 0°C bis 50°C. Das gebildete Xanthogenat kann z.B. mit Alkylhalogeniden, z.B. Methyljodid, Alkylsulfaten (z.B. Dimethylsulfat) bei 0° bis 120°C zu den entsprechenden Xanthogensäureestern alkyliert werden. Als Lösungsmittel können beispielsweise Alkohole oder eines der oben genannten allgemeinen Lösungsmittel dienen. Nach einer etwas besseren Methode überführt man Soraphen mit einem Halogenthioameisensäureester wie Chlorthioameisensäurephenylester in trockenem Kohlenwasserstoff oder Chlorkohlenwasserstoff als Lösungsmittel in Gegenwart von Pyridin in das 5-O-Thionocarbonat.

Daraus, wie auch aus den entsprechenden Halogeniden, lässt sich die Desoxyverbindung durch Umsetzung mit Triaryl- bzw. Trialkyl-Zinnhydriden (z.B. Tributylzinnhydrid) in inerten Lösungsmitteln (z.B. Benzol) unter Inertgasatmosphäre bei Temperaturen zwischen 0°C und 120°C, gegebenenfalls unter Verwendung von Radikalstartern wie Dibenzoylperoxid, Azo-bis-isobutyronitril oder Belichtung, erhalten. [Houben Weyl. "Methoden der organ. Chemie", Band 9, 1d, S. 341, 388ff].

Die Verätherung der 5-OH-Gruppe erfolgt als solche oder in der Alkali-Alkoholatform durch organische Halogenide oder Sulfate. Liegt die freie OH-Gruppe vor, arbeitet man vorzugsweise in Gegenwart von säurebindenden Mitteln wie Alkali(hydrogen)carbonat oder tert. Aminen wie Trialkylamin oder Pyridin. Als Halogenide kommen bevorzugt Bromide und Jodide wie Methyljodid, Propargylbromid, Allylbromid, Cyclopropylbromid, Cyclopentylbromid, Phenyljodid, p-Nitrophenylbromid, aber auch Chlormethylmethylether, vorteilhaft in Gegenwart katalytischer Mengen NaJ in Frage. Niederalkylgruppen lassen sich vorteilhaft als Alkylsulfate einführen, z.B. Dimethylsulfat, Diethylsulfat und andere.

Als Lösungsmittel kommen beispielsweise Aceton, Dimethylformamid, Pyridin oder etherartige Verbindungen wie Tetrahydrofuran, Diethylether, 1,2-Dimethoxyethan in Frage. Die Reaktionstemperaturen liegen bei 0° bis 100°C.

Sind im Molekül oder im Reaktanden störende funktionelle Gruppen wie OH, $NH_2$, -COOH, so können diese, wie bereits oben erwähnt, durch Acetylierung oder Einführung anderer Schutzgruppen einleitend maskiert werden [T.W. Green "Protective Groups in Organic Synthesis", J. Wiley & Sons, 1981 (New York)].

Die Aufzählung aller vorgenannten Methoden ist nicht limitierend. Gewünschtenfalls können die Endprodukte auf übliche Art und Weise gereinigt werden, z.B. durch Waschen, Digerieren, Extraktion, Umkristallisation, Chromatographie usw..

Die beschriebenen Herstellungsverfahren einschliesslich aller Teilschritte sind ein Bestandteil der vorliegenden Erfindung.

9,10-Dihydro-Soraphen A lässt sich auf Soraphen A oder einem in 5-Position geschützten Derivat durch Hydrierung beispielsweise mit homogenen Hydrierkatalysatoren auf der Basis von Uebergangsmetallkomplexen (z.B. Rhodium- oder Iridiumkomplexen) erhalten.

Es ist zu beachten, dass die macrocyclischen Soraphen der Formel I normalerweise in der angegebenen Hemiacetalform vorliegen, diese Form jedoch eine reversible Ringöffnung nach dem Schema

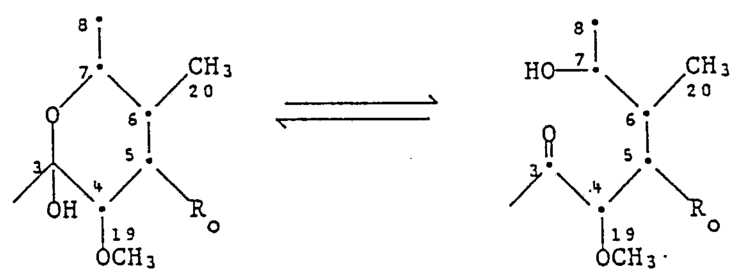

erfahren kann. Je nach Herstellungs- bzw. nach Aufarbeitungsmethodik fallen, abhängig vom pH-Wert und vom Lösungsmittel, die Soraphene in der einen oder anderen Form oder als Gemisch beider Formen an. Charakteristisch für die Ringöffnung ist die Verschiebung des $^{13}$C-NMR-Signals in der 3-Position und die der $^{1}$H-NMR-Signale in bestimmten anderen Positionen. Beim Soraphen A werden beispielsweise folgende Veränderungen beobachtet: $^{13}$C-NMR($CDCL_3$, δ in ppm) 99,5 ⟶203,1(3-C).$^{1}$H-NMR($CDCl_3$, δ in ppm): 3,14⟶3,72(2-H); 3,18⟶4,5(4-H); 3,83⟶3,16 (7-H); 5,86⟶5,7 (17-H). Aehnliche Verschiebungen werden auch bei den hierin genannten Soraphen-Derivaten der Formel I beobachtet. Die Formel I vorliegender Erfindung umfasst grundsätzlich die bei niedrigen pH-Werten bevorzugte 3-Hemiacetalform und die geöffnete 3-Keto-7-hydroxyform sowie alle unter die Formel I fallenden möglichen Stereoisomeren.

Es wurde gefunden, dass Verbindungen der Formel I ein für praktische Bedürfnisse sehr günstiges biozides Spektrum gegen phytopathogene Mikroorganismen, insbesondere gegen Pilze, aufweisen. Sie besitzen sehr vorteilhafte kurative, systemische und insbesondere präventive Eigenschaften und werden zum Schutz von zahlreichen Kulturpflanzen eingesetzt. Mit den Wirkstoffen der Formel I können an Pflanzen oder Pflanzenteilen (Früchte, Blüten, Laubwerk, Stengel, Knollen, Wurzeln) von unterschiedlichen Nutzkulturen die auftretenden Schädlinge eingedämmt oder vernichtet werden, wobei auch später zuwachsende Pflanzenteile

vor phytopathogenen Mikroorganismen verschont bleiben.

Als Mikrobizide sind die Wirkstoffe der Formel I z.B. gegen die den folgenden Klassen angehörenden phytopathogenen Pilze wirksam: Fungi imperfecti (z.B. insbesondere Botrytis, ferner Pyricularia, Helminthosporium, Fusarium, Septoria, Cercospora und Alternaria); Basidiomyceten (z.B. Rhizoctonia, Hemileia, Puccinia). Darüber hinaus wirken sie gegen die Klasse der Ascomyceten (z.B. insbesondere Venturia und Erysiphe, ferner Podosphaera, Monilinia, Uncinula) und der Oomyceten (z.B. Phytophthora, Plasmopara). Die Verbindungen der Formel I können ferner als Beizmittel zur Behandlung von Saatgut (Früchte, Knollen, Körner) und Pflanzenstecklingen zum Schutz vor Pilzinfektionen sowie gegen im Erdboden auftretende phytopathogene Pilze eingesetzt werden.

Die Erfindung betrifft auch die Mittel, die als Wirkstoffkomponente Verbindungen der Formel I in einer der möglichen stereoisomeren Formen enthalten, insbesondere pflanzenschützende Mittel, sowie deren Verwendung auf dem Agrarsektor oder verwandten Gebieten.

Eingeschlossen ist auch ein Verfahren zur Behandlung von Pflanzen, das sich durch Applikation der neuen Verbindungen der Formel I bzw. der entsprechenden neuen Mittel auszeichnet.

Als Zielkulturen für den hierin offenbarten pflanzenschützenden Einsatz gelten im Rahmen dieser Erfindung beispielsweise folgende Pflanzenarten: Getreide (Weizen, Gerste, Roggen, Hafer, Reis, Mais, Sorghum und verwandte Species); Rüben (Zucker- und Futterrüben); Kern-, Stein- und Beerenobst (Aepfel, Birnen, Pflaumen, Pfirsiche, Mandeln, Kirschen, Erdbeeren, Himbeeren und Brombeeren); Hülsenfrüchte (Bohnen, Linsen, Erbsen, Soja); Oelkulturen (Raps, Senf, Mohn, Oliven, Sonnenblumen, Kokos, Rizinus, Kakao, Erdnüsse); Gurkengewächse (Kürbis, Gurken, Melonen); Fasergewächse (Baumwolle, Flachs, Hanf, Jute); Citrusfrüchte (Orangen, Zitronen, Pampelmusen, Mandarinen); Gemüsesorten (Spinat, Kopfsalat, Spargel, Kohlarten, Möhren, Zwiebeln, Tomaten, Kartoffeln, Paprika); Lorbeergewächse (Avocado, Cinnamomum, Kampfer) oder Pflanzen wie Tabak, Nüsse, Kaffee, Ananas, Zuckerrohr, Tee, Pfeffer, Weinreben, Hopfen, Bananen- und Naturkautschukgewächse sowie Zierpflanzen (Compositen). Diese Aufzählung stellt keine Limitierung dar.

Wirkstoffe der Formel I werden üblicherweise in Form von Zusammensetzungen verwendet und können gleichzeitig oder nacheinander mit weiteren Wirkstoffen auf die zu behandelnde Fläche oder Pflanze gegeben werden. Diese weiteren Wirkstoffe können sowohl Düngemittel, Spurenelemente-Vermittler oder andere das Pflanzenwachstum beeinflussende Präparate sein. Es können dabei auch selektive Herbizide sowie Insektizide, Fungizide, Bakterizide, Nematizide, Molluskizide oder Gemische mehrere dieser Präparate zusammen mit gegebenenfalls weiteren in der Formulierungstechnik üblichen Trägerstoffen, Tensiden oder anderen applikationsfördernden Zusätzen Verwendung finden.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln.

Ein bevorzugtes Verfahren zum Aufbringen eines Wirkstoffs der Formel I bzw. eines agrochemischen Mittels, das mindestens einen dieser Wirkstoffe enthält, ist das Aufbringen auf das Blattwerk (Blattapplikation). Applikationsfrequenz und Aufwandmenge richten sich dabei nach dem Befallsdruck des betreffenden Erregers. Die Wirkstoffe der Formel I können aber auch über den Erdboden durch das Wurzelwerk in die Pflanze gelangen (systemische Wirkung), indem man den Standort der Pflanze mit einer flüssigen Zubereitung tränkt oder die Substanzen in fester Form in den Boden einbringt, z.B. in Form von Granulat (Bodenapplikation).

Verbindungen der Formel I können auch auf Samenkörner aufgebracht werden (Coating), indem man die Körner entweder in einer flüssigen Zubereitung des Wirkstoffs tränkt oder sie mit einer festen Zubereitung beschichtet.

Die Verbindungen der Formel I werden dabei in unveränderter Form oder vorzugsweise zusammen mit den in der Formulierungstechnik üblichen Hilfsmitteln eingesetzt. Dafür werden sie zweckmässigerweise z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt. Günstige Aufwandmengen liegen im allgemeinen bei 10 g bis 500 g Aktivsubstanz (AS) pro Hektar, bevorzugt bei 50 g bis 500 g AS/ha.

Die Formulierungen d.h. die den Wirkstoff der Formel I und einen festen oder flüssigen Zusatzstoff enthaltenden Mittel werden in bekannter Weise hergestellt.

Als Lösungsmittel kommen in Frage: Aromatische und aliphatische Kohlenwasserstoffe, wie z.B. Xylolgemische, Cyclohexan oder Paraffine; dann Alkohole und Glykole sowie deren Ether und Ester, wie Ethanol, Ethylenglykol, Ethylenglykolmonomethyl- oder -ethylether, Essigsäureester; Ketone wie Cyclohexanon, stark polare Lösungsmittel, wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorilonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht-sorptive Trägermateria lien, z.B. Calcit oder Sand, in

Frage. Darüber hinaus kann eine Vielzahl von vorgranulierten Materialien anorganischer Natur wie insbesondere Dolomit oder zerkleinerte Pflanzenrückstände verwendet werden.

Als oberflächenaktive Verbindungen kommen je nach Art des zu formulierenden Wirkstoffs der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen, als auch wasserlösliche synthetische oberflächenaktive Verbindungen sein.

Häufiger werden jedoch sog. synthetische Tenside verwendet, insbesondere Alkansulfonate, Fettalkoholsulfate, sulfonierte Benzimidazolderivate oder Alkylsulfonate.

Als nichtionische Tenside kommen Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Als weitere Beispiele nichtionischer Tenside seien Nonylphenolpolyethoxyethanole, Ricinusölpolyglykolether, Polypropylen-Polyethylenoxydaddukte, Tributylphenoxypolyethylenethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Weitere in der Formulierungstechnik gebräuchlichen Tenside sind dem Fachmann bekannt oder können der einschlägigen Fachliteratur entnommen werden.

Die agrochemischen Zubereitungen enthalten in der Regel 0,1 bis 95 % Wirkstoff der Formel I, 99,9 bis 5 % eines festen oder flüssigen Zusatzstoffes und 0 bis 25 % eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Die nachfolgenden Beispiele dienen zur näheren Erläuterung der Erfindung, ohen dieselbe einzuschränken. (Es bedeuten: h = Stunde. PSC = präparative Schicht-Chromatographie. DC = Dünnschichtchromatographie. RT = Raumtemperatur.).

## 1. Herstellungsbeispiele

### H-1. Herstellung von Soraphen A-5-formiat (Verb. Nr. 1)

50.0 mg (0.096 mmol) Soraphen A und 11.7 mg (0.096 mmol) 4-Dimethylamino-pyridin (DMAP) werden in 2 ml trockenem Dichlormethan gelöst und mit 8.7 µl (10.7 mg, 0.233 mmol) Ameisensäure und 66,6 µl (48.4 mg, 0.478 mmol) Triethylamin versetzt. Die Reaktionsmischung wird auf 10° C gekühlt, mit 18.5 µl (20.0 mg, 0.196 mmol) Essigsäureanhydrid versetzt und 30 min. gerührt. Das Eisbad wird entfernt und man lässt noch 30 min. bei Raumtemperatur rühren. Dann wird das Reaktionsgemisch mit einigen Tropfen Methanol versetzt und eingeengt. Der Rückstand wird mit Wasser versetzt und mehrmals mit Ethylacetat extrahiert. Die vereinigten organischen Phasen werden nacheinander mit 1N-HCl (pH 0), 5 %iger Natriumhydrogencarbonatlösung (pH 8) und mit konz. Kochsalzlösung (pH 6) gewaschen. Dann wird über Natriumsulfat getrocknet und am Rotationsverdampfer eingeengt. Man erhält 50.7 mg (0.092 mmol, 96 %) NMR-spektroskopisch einheitliches Produkt.

### H-2. Herstellung von Soraphen A-5-propionat (Verb. Nr. 6)

50 mg (0.096 mmol) Soraphen A werden in 0.5 ml trockenem Pyridin gelöst und mit 28 µl (29.6 mg, 0.320 mmol) Propionsäurechlorid, sowie 12 mg (0.10 mmol) DMAP versetzt. Nach 20 h Rühren bei Raumtemperatur wird die Lösung weitgehend eingeengt, mit Wasser versetzt und mit Ethylacetat extrahiert. Die vereinigten organischen Phasen werden mit 5 %iger $NaHCO_3$-Lösung und gesättigter Kochsalzlösung gewaschen, über Natriumsulfat getrocknet und am Rotationsverdampfer eingeengt. Man erhält 53 mg eines Rohproduktes, das mittels PSC (Merck, Kieselgel 60, Laufmittel: Dichlormethan/Aceton 90:10) gereinigt wird.

Man erhält 23 mg (0.040 mmol, 42 %) des Produktes.

### H-3. Herstellung von Soraphen A-5-carbamat (Verb. Nr. 139)

30 mg (0.06 mmol) Soraphen A werden in 0.5 ml trockenem Dichlormethan gelöst und mit 14 µl (22 mg, 0.12 mmol) Trichloracetylisocyanat versetzt. Nach 10 min wird die Reaktionsmischung über neutrales Aluminiumoxid filtriert, wobei die Trichloracetylgruppe entfernt wird, und mit Dichlormethan eluiert.

Man erhält 32.0 mg des Rohproduktes, das mittels PSC (Kieselgel $60F_{254}$, Lm: Dichlormethan/Methanol/Toluol 95:5:1) gereinigt wird. Ausbeute: 29.0 mg (0.05 mmol, 83 %) weisser Feststoff.

### H-4. Herstellung von 5-Methoxy-Soraphen A (Verb. Nr. 92)

16 mg (0.03 mmol) Soraphen A werden in 0.5 ml Dimethylsulfoxid (DMSO) gelöst und mit 10 mg (0.18 mmol) KOH, sowie 50 µl (104 mg, 0.80 mmol) $CH_3Cl$ versetzt. Man lässt 5 h bei Raumtemperatur rühren und engt die Reaktionsmischung dann weitgehend ein. Der Rückstand wird mit einer Pufferlösung (pH 7) versetzt und mit Ethylacetat extrahiert. Die organische Phase wird mit konzentrierter NaCl-Lsg. gewaschen, über $Na_2SO_4$

getrocknet und am Rotationsverdampfer eingeengt. Man erhält 12 mg des Rohproduktes, das mittels PSC (Kieselgel 60, Lm: $CH_2Cl_2$/Aceton 90:10) gereinigt wird. Die unpolare Zone wird eluiert. Ausbeute: 4.5 mg (0.008 mmol, 26 %) farbloses Oel.

H-5. Herstellung von Soraphen A-5-mesylat (Verb. Nr. 74).

102 mg (0.195 mmol) Soraphen A werden in 5 ml trockenem Dichlormethan gelöst, nacheinander mit 67 µl (49 mg, 0.484 mmol) $Et_3N$ und 19 µl (28 mg, 0.244 mmol) Mesylchlorid versetzt und 1 h bei Raumtemperatur gerührt. Die Reaktionsmischung wird dann mit 1N-HCl versetzt und mit $CH_2Cl_2$ extrahiert. Die vereinigten organischen Phasen werden mit 5 %iger $NaHCO_3$-Lösung und konz. NaCl-Lösung gewaschen, über $CaCl_2$ getrocknet und am Rotationsverdampfer eingeengt.

Man erhält 116 mg (0.193 mmol, 99 %) des NMR-spektroskopisch einheitlichen Rohproduktes, das mittels PSC (Merck, Kieselgel 60, Laufmittel: $CH_2Cl_2$/Aceton 90:10) zur Charakterisierung gereinigt werden kann.

H-6. Herstellung von 5-tert. Butyldimethylsilyloxy-Soraphen A (Verb. Nr. 73)

20 mg (0.038 mmol) Soraphen A werden in 1 ml Dimethylformamid (DMF) gelöst und mit ca. 50 mg (0.33 mmol) Imidazol, ca. 50 mg (3.40 mmol) t-Butyldimethylsilylchlorid, sowie 5 mg (0.04 mmol) DMAP versetzt. Man lässt 2 Tage bei Raumtemperatur rühren und engt die Reaktionsmischung dann weitgehend ein. Der Rückstand wird mit Pufferlösung (pH 7) versetzt und mit Ethylacetat extrahiert. Die organische Phase wird mit konzentrierter NaCl-Lösung gewaschen, über $Na_2SO_4$ getrocknet und am Rotationsverdampfer eingeengt. Man erhält 25.5 mg des Rohproduktes, das mittels präparativer Mitteldruckchromatographie (stationäre Phase: Lichroprep Si 60, 25 - 40 µm, Merck Art. 9390; Lm: $CH_2Cl_2$/t-Butylmethylether 96:4, Fluss 26 ml/min, Druck: 12 bar) gereinigt wird. Man erhält 10 mg (0.016 mmol, 42 %) farbloses Oel.

H-7. Herstellung von Soraphen B-5,11-diacetat (Verb. Nr. 10)

29 mg (0.057 mmol) Soraphen B werden in 0.5 mol Pyridin gelöst und mit 46 µl (50 mg, 0.49 mmol) Essigsäureanhydrid versetzt. Man lässt über Nacht bei Raumtemperatur rühren und engt die Reaktionsmischung dann weitgehend ein. Der Rückstand wird mit Wasser versetzt und mit Ethylacetat extrahiert. Die vereinigten organischen Phasen werden nacheinander mit 1N-HCl, 5 %iger $NaHCO_3$-Lösung und gesättigter NaCl-Lösung gewaschen, über $NaSO_4$-Lösung getrocknet und am Rotationsverdampfer eingeengt. Man erhält 28 mg des Rohproduktes, das mittels PSC (Kieselgel 60, Lm: $CH_2Cl_2$/Aceton 93:7, Elution der unpolaren Zone) gereinigt wird. Ausbeute: 12 mg (0.020 mmol, 35 %) farbloses Oel.

H-8. Herstellung von Soraphen B-5-acetat (Verb. Nr. 12) und Soraphen B-11-acetat (Verb. Nr. 165)

18 mg (0.035 mmol) Soraphen B werden in 0.1 ml Pyridin gelöst und mit 30 µl (32 mg, 0.32 mmol) Essigsäureanhydrid versetzt. Nach 3stündigem Rühren bei Raumtemperatur wird die Reaktionsmischung weitgehend eingeengt, mit 1N-HCl versetzt und mit Ethylacetat extrahiert. Die vereinigten, organischen Phasen werden mit konz. NaCl-Lösung gewaschen, über $Na_2SO_4$ getrocknet und am Rotationsverdampfer eingeengt. Das Rohprodukt wird mittels PSC (Merck, Kieselgel 60, Lm: $CH_2Cl_2$/Aceton 90:10) gereinigt.

H-9. Herstellung von Soraphen A-5-t-butylcarbonat (Verb. Nr. 170)

50 mg (0.1 mmol) Soraphen A werden in 1 ml Dichlormethan gelöst und mit 70 µl (51 mg, 0.23 mmol) Triethylamin versetzt. Zu der, auf -10° C gekühlten, Lösung gibt man 44 mg (0.20 mmol) Di-t-butylpyrocarbonat. Nach 2stündigem Rühren bei -10° C ist die Reaktion beendet. Es wird mit 1N HCl versetzt und mit Ethylacetat extrahiert. Die vereinigten organischen Phasen werden mit 5 %iger $NaHCO_3$-Lösung, sowie gesättigter Kochsalzlösung gewaschen, über Natriumsulfat getrocknet und am Rotationsverdampfer eingeengt. Es werden 45 mg des Rohproduktes erhalten, das mittels PSC (Merck, Kieselgel 60, Laufmittel: Dichlormethan/Aceton 95:5) gereinigt wird.

Es werden 26 mg (0.042 mmol, 40 %) Produkt erhalten.

H-10. Herstellung von 5-O-Tetrahydropyranyl-Soraphen A als Isomer I und als Isomer II (Verb. Nr. 131)

200 mg (0.38 mmol) Soraphen A, gelöst in 5 ml Dichlormethan, werden mit 180 µl (166 mg, 1.97 mmol) 3,4-Dihydro-2H-pyran und 1 mg p-Toluolsulfonsäure-Hydrat versetzt. Nach 15minütigem Rühren bei Raumtemperatur wird die Reaktionslösung mit 5 %iger $NaHCO_3$-Lösung versetzt und 2mal mit Ethylacetat extrahiert. Die vereinigten organischen Phasen werden mit gesättigter NaCl-Lösung gewaschen, über $Na_2SO_4$ getrocknet und am Rotationsverdampfer eingeengt.

Es werden 215 mg (90 %) des 5-O-THP-Ethers als 1:1-Isomerengemisch erhalten. Die Isomeren können mittels MPLC (Merck Lichroprep Si 60, 15 - 25 µm, Art. 9336, Laufmittel: Dichlormethan/Methanol, 995:5, Detektion bei 254 nm) getrennt werden. Es eluiert zunächst Isomer I, dann Isomer II.

Tabelle 1

| Nr. | $R_f$(Lm)* | MS(EI) | $^1$H-NMR-Daten ausgewählter Signale ($CDCl_3$, δ in ppm) | | | | | | |
|---|---|---|---|---|---|---|---|---|---|
| | | $M^+$ | 2-H | 4-H | 5-H | 6-H | 8-H | 3-OH | $R/R_o$ |
| 1 | 0.68 (1) | 548 | 3.14 | 3.14 | 5.29 | 1.88 | 2.45 | 3.92 | 8.12 (s,1H) |
| 4 | 0.58 (1) | 562 | 3.12 | 3.10 | 5.05 | 1.82 | 2.45 | 3.74 | 2.12 (s,3H) |
| 5 | - | 616 | 3.12 | 3.21 | 5.12 | 1.94 | 2.46 | 3.72 | - |
| 6 | 0.73 (1) | 576 | 3.14 | 3.10 | 5.06 | 1.82 | 1.96 | 3.72 | 1.16 (t,3H), 2.39 (q,2H) |
| 7 | 0.63 (1) | 590 | 3.11 | 3.09 | 5.06 | 1.82 | 2.45 | 3.72 | 0.98 (t,3H), 1.67 (m,2H), 2.35 (t,2H) |
| 8 | 0.46 (2) | 590 | 3.12 | 3.08 | 5.03 | 1.82 | 2.46 | 3.70 | 1.19 (d,3H), 1.20 (d,3H), 2.60 (qq,1H) |
| 15 | 0.51 (2) | 624 | 3.18 | 3.26 | 5.30 | 2.02 | 2.52 | 3.81 | 7.48 (pt,2H), 7.59(pt,1H), 8.08 (pd,2H) |
| 139 | 0.55 (3) | 563 | 3.13 | 3.15 | 4.96 | 1.87 | 2.45 | 3.82 | 6.52 (s,b), 6.67 (s,b), $NH_2$ |
| 141 | 0.31 (2) | 639 | 3.15 | 3.22 | 5.07 | 1.93 | 2.47 | 3.80 | 6.77 (b,N-H), 7.07 (m,1H), 7.32 (m,2H), 7.43 (m,2H) |
| 67 | - | 592 | 3.03 | 2.96 | 4.11 | 1.64 | 2.50 | 5.29 | 0.18 (s,9H) |
| 92 | 0.61 (1) | 534 | 3.04 | 3.11 | 3.60 | 1.97 | 2.50 | 4.89 | 3.43 (s,3H) |
| 74 | 0.63 (1) | 598 | 3.15 | 3.34 | 4.91 | 2.08 | 2.45 | 3.80 | 3.11 (s,3H) |
| 75 | 0.61 (1) | - | 3.06 | 3.15 | 4.67 | 1.83 | 2.35 | 3.78 | 2.46 (s,3H), 7.37 (pd,2H), 7.84 (pd,2H) |
| 73 | 0.68 (1) | 634 | 3.02 | 2.98 | 4.14 | 1.74 | 2.51 | 5.17 | 0.14 (s,3H), 0.15 (s,3H), 0.92 (s,9H) |
| 25 | 0.81 (1) | - | 3.13 | 3.23 | 5.09 | 1.99 | 2.46 | 3.70 | - |
| 12 | 0.37 (1) | - | 3.10 | 3.07 | 5.04 | ** | ** | 3.84 | 2.13 (s,3H) |
| 10 | 0.45 (2) | - | 3.11 | 3.06 | 5.04 | ** | ** | 3.91 | 2.08 (s,3H), 2.13 (s,3H) |
| 13 | 0.35 (1) | 578 | 3.10 | 3.05 | 5.06 | ** | ** | 3.83 | 0.98 (t,3H), 1.69 (m,2H), 2.36 (t,2H) |
| 11 | - | 648 | 3.11 | 3.06 | 5.04 | ** | 2.12 | 3.89 | 5.06 (H-11), 2.34 (m,4H) |
| 14 | 0.39 (1) | 612 | 3.16 | 3.21 | 5.29 | ** | 2.17 | 3.92 | 7.49 (pt,2H), 7.59 (pt,1H), 8.09 (pd,2H) |
| 165 | 0.46 (1) | - | 3.09 | 3.14 | 3.97 | ** | ** | 4.46 | 2.08 (s,3H), 5.08 (m, 1H) |

* Lm 1: Dichlormethan/Aceton 90:10
Lm 2: Dichlormethan/Aceton 95:5
Lm 3: Dichlormethan/Aceton 75:25
** Signal verdeckt

Tabelle 1 (Fortsetzung)

| Nr. | $R_f$(Lm)* | MS(EI) $M^+$ | $^1$H-NMR-Daten ausgewählter Signale ($CDCl_3$, δ in ppm) 2-H | 4-H | 5-H | 6-H | 8-H | 3-OH | $R/R_o$ |
|---|---|---|---|---|---|---|---|---|---|
| 170 | 0.66 (1) | 620 | 3.12 | 3.14 | 4.88 | 1.89 | 2.47 | 3.88 | 1.52 (s, 9H) |
| 149 | 0.56 (1) | 578 | 3.14 | 3.18 | 4.91 | 1.97 | 2.47 | 3.90 | 3.82 (s, 3H) |
| 22 | 0.69 (1) | 692 | 3.19 | 3.25 | 5.32 | 2.00 | 2.51 | 3.85 | 7.74 (m, 2H), 8.20 (m, 2H) |
| 171 | 0.49 (2) | 604 | 3.12 | 3.08 | 5.00 | 1.79 | 2.47 | 3.67 | 1.22 (s, 9H) |
| 55 | 0.32 (4) | | 3.12 | 3.11 | 5.05 | 1.84 | 2.44 | - | 2.64 (m, 4H) |
| 49 | 0.64 (1) | 602 | 3.14 | 3.13 | 5.11 | 1.88 | 2.47 | 3.77 | 6.91 (m, 1H) |
| 32 | 0.57 (1) | 574 | 3.14 | 3.15 | 5.14 | 1.88 | 2.47 | 3.76 | 5.88 (m, 1H), 6.20 (m, 1H), 6.47 (m, 1H) |
| 47 | 0.36 (1) | 592 | 3.15 | 3.12 | 5.12 | 1.86 | 2.45 | 3.71 | 4.08 (m, 2H) |
| 50 | 0.39 (1) | | 3.10 | 3.09 | 5.09 | ** | 2.12 | 3.92 | 4.05 (m, 2H), 4.10 (2H), 5.17 (m, 2H) |
| 131 | 0.44 (1) | 604 | 3.07 | 3.18 | 4.17 | 1.96 | 2.51 | 4.85 | 1.57 (m, 4H), 1.77 (m, 2H), 3.56 (m, 1H), 3.86 (m, 1H), 4.70 (m, 1H) |
| 131 | 0.42 (1) | 604 | 3.06 | 3.02 | 4.07 | 1.99 | 2.51 | 4.82 | 1.62 (m, 4H), 1.75 (m, 2H), 3.55 (m, 1H), 3.81 (m, 1H), 4.69 (m, 1H) |
| 168 | 0.33 (1) | | 3.09 | 3.12 | 3.98 | 1.86 | 2.18 | - | 4.05 (m, 2H), 5.20 (m, 1H, 11-H), 3.81 (m, 1H), 4.69 (m, 1H) |

* Lm 1: Dichlormethan/Aceton 90:10
Lm 2: Dichlormethan/Aceton 95:5
Lm 3: Dichlormethan/Aceton 75:25
Lm 4: Dichlormethan/Aceton/Methanol 78:20:2

** Signal verdeckt

Tabelle 2

| Nr. | $^{13}C$-NMR-Daten ausgewählter Signale ($CDCl_3$, δ in ppm) | | |
|---|---|---|---|
| | Carbonyl-C | C-4/C-5/C-7/C-17 | $R/R_o$ |
| 1 | 160.2/170.8 | 70.0/72.4/74.5/74.8* | - |
| 4 | 170.4/171.0 | 70.4/72.4/74.1/74.7* | 21.5 |
| 5 | 170.8/- | 71.9/73.4/74.5/74.7* | - |
| 6 | 171.0/173.9 | 70.3/72.5/74.1/74.8* | 9.0, 35.9 |
| 7 | 171.0/173.0 | 70.3/72.6/74.2/74.9* | 13.7, 35.9, 36.5 |
| 8. | 171.1/176.5 | 70.1/72.5/74.1/74.6* | 18.8, 18.9, 34.0 |
| 15 | 166.0/171.1 | 70.7/72.7/74.5/74.9* | 129.8, 130.4, 133.2 |
| 139 | 155.9/171.0 | 71.0/72.6/74.2/75.0* | - |
| 141 | 152.8/171.0 | 71.0/72.7/74.3/75.9* | 118.7, 123.6, 129.1, 137.7 |
| 67 | - | - | - |
| 92 | 171.1/- | 72.2/72.5/74.4/79.1* | 57.7 |
| 74 | 170.7/- | 71.8/74.6/75.2/76.3* | 39.1 |
| 75 | 170.9/- | 71.6/74.5/75.4/77.5* | 21.7, 127.9, 129.9, 134.7, 145.1 |
| 73 | 171.2/- | 70.8/71.8/72.0/77.3* | 5.0, 25.9 |
| 25 | 161.5/170.9 | 72.1/74.0/74.6/75.6* | 89.9 |

* Zuordnung offen

Tabelle 2 (Fortsetzung)

| Nr. | $^{13}$C-NMR-Daten ausgewählter Signale ($CDCl_3$, δ in ppm) | | |
|---|---|---|---|
| | Carbonyl-C | C-4/C-5/C-7/C-17 | R/$R_o$ |
| 170 | 153.0/171.1 | 72.1/73.2/73.9/75.1* | 27.9, 82.9 |
| 149 | 155.2/171.0 | 72.1/74.1/74.4/74.8* | 54.9 |
| 22 | 164.8/170.9 | 71.2/72.7/74.7/74.9* | 125.5, 125.6, 130.3, 133.7 |
| 171 | 171.2/177.9 | 70.0/72.6/74.2/74.6* | 27.1, 38.9 |
| 55 | 170.9/171.9 | 70.8/72.8/74.0/74.9* | 32.3, 35.1 |
| 49 | 167.3/171.2 | 70.2/72.7/74.2/74.9* | 12.1, 14.6, 138.2 |
| 32 | 165.6/171.1 | 70.5/72.5/74.3/74.8* | 128.5, 131.6 |
| 47 | 169.8/170.9 | 70.9/72.4/74.3/74.6* | 69.9 |
| 50 | 169.9/170.1/171.6 | 69.8/71.2/74.7/76.1* | 57.7, 59.4, 69.9 |
| 131 | 171.2 | 72.1/72.1/73.2/77.3* | 19.6, 25.2, 31.2, 63.2, 97.7 |
| 131 | 171.1 | 72.3/72.5/74.5/75.1* | 19.3, 25.2, 31.1, 62.9, 98.8 |

* Zuordnung offen

H-11. Herstellung von 5-O-(2,4-Dinitrophenyl)-Soraphen A (Verb. Nr. 172)

25 mg (0.1 mmol) Soraphen A und 30 mg 2,4-Dinitro-1-fluorbenzol (2 eq.) werden in 1 ml trockenem DMF gelöst und die Lösung wird zu 8 mg Natriumhydrid gespritzt. Nach 78 h Rühren bei RT zeigt ein Dünnschichtchromatogramm (DC) (Laufmittel Dichlormethan/Aceton, 9:1, v/v; Edukt Rf 0.3, Produkt Rf 0.6) eine vollständige Umsetzung an.

Die Lösung wird mit 10 ml 1 N Chlorwasserstoffsäure (= HCl) verdünnt, zweimal mit Ethylacetat extrahiert, das Lösungsmittel abdestilliert und das Produkt mit Hilfe der PSC gereinigt (Kieselgel Si 60, 1 mm, Laufmittel Dichlormethan/Aceton, 9:1, v/v; Rf 0.5).

Ausbeute: 23 g = 34 % der Theorie.

$^1$H-NMR ($CDCl_3$): δ = 2.12 (m, 1 H, H-6); 2.50 (m, 1 H, H-8); 3.09 (q, 1 H, H-2); 3.24 (d, 1 H, H-4); 4.35 (s, 1 H, 3-OH); 4.87 (m, 1 H, H-5);
7.27, 8.47, 8.86 (2,4-Dinitrophenyl).

$^{13}$C-NMR ($CDCl_3$): δ = 31.90 d, 35.09 d (C-6, C-8); 98;34 s (C-3); 170.75 s (C-1);
115.06 d, 122.86 d, 129.26 d, 139.80 s, 140.65 s, 154.95 s (2,4-Dinitrophenyl).

IR (Film): ν = 3544, 2933, 2885, 2829, 1731, 1608, 1538, 1488, 1483, 1347, 1276, 1149, 1093, 977, 836, 745, 701 $cm^{-1}$.
UV (Methanol): $\lambda_{max}$ (1g ε) = 289 nm (4.11)
MS (70 eV): m/e (%) = 686 (0.4, $M^+$), 654 (0.3), 497 (2), 479 (1), 465 (1), 297 (1), 281 (2), 189 (25), 157 (94), 91 (100)

| | | |
|---|---|---|
| Hochauflösung: | $C_{35}H_{46}N_2O_{12}$ | Ber.: 686.3050 |
| | | Gef.: 686.3065 $M^+$ |

H-12. Herstellung von 5-O-Allyl-Soraphen A (Verb. Nr. 84)

52 mg (0.1 mmol) Soraphen A und 60 mg Allylbromid (5 eq) werden in 0.7 ml trockenem DMF gelöst, und die Lösung wird zu 22 mg (2 eq) Kalium-tert-butylat gespritzt. Nach 15 min Rühren bei RT zeigt ein DC (Laufmittel Dichlormethan/Aceton, 9:1, v/v; Edukt Rf 0.3, Produkt Rf 0.6) eine vollständige Umsetzung an.

Die Lösung wird mit 10 ml 1 N HCl verdünnt, zweimal mit Ethylacetat extrahiert, das Lösungsmittel abdestilliert und das Produkt mit Hilfe der PSC gereinigt (Kieselgel Si 60, 1 mm, Laufmittel Dichlormethan/Aceton, 9:1, v/v; Rf 0.5).
Ausbeute: 14.8 mg = 27 % der Theorie.
$^1$H-NMR-($CDCl_3$): δ = 1.92 (m, 1 H, H-6); 2.50 (m, 1 H, H-8); 3.03 (q, 1 H, H-2); 3.10 (d, 1 H, H-4); 3.78 (m, 1 H, H-5); 4.97 (s, 3-OH);
4.02, 4.14, 5.24, 5.30, 5.89 (Allyl).
$^{13}$C-NMR ($CDCl_3$): δ = 31.67 d, 35.23 d (C-6, C-8); 99.07 s (C-3); 171.07 s (C-1);
71.16 t, 118.08 t, 133.97 d (Allyl).
IR (Film): ν = 3487, 2960, 2933, 2871, 1733, 1463, 1380, 1260, 1177, 1096, 1071, 990, 747 $cm^{-1}$.
UV (Methanol): $\lambda_{max}$ (1 g ε) = 204 nm (4.09)
MS (70 eV): m/e (%) = 560 (5, $M^+$), 542 (3), 528 (2), 502 (2), 470 (4), 295 (5), 215 (10), 189 (32), 157 (94), 71 (100).

| | | |
|---|---|---|
| Hochauflösung: | $C_{32}H_{48}O_8$ | Ber.: 560.3369 |
| | | Gef.: 560.3352 $M^+$ |

H-13. Herstellung des 5-O-Glycinesters von Soraphen A (Verb. Nr. 53)

a) Herstellung des Zwischenprodukts N-(BOC)-glycinester

52 mg (0.1 mmol) Soraphen A, 19 mg N-tert. Butyloxycarbonyl-Glycin (= N-BOC-Glycin) (1.1 eq), 26 mg Dicyclohexylcarbodiimid (= DCC) (1.2 eq) und 1.2 mg N,N-Dimethylaminopyridin (= DMAP) werden in 1 ml trockenem Dichlormethan gelöst und 1 h bei RT gerührt. Ein DC (Dichlormethan/Aceton, 9:1, v/v; Edukt Rf 0.3, Produkt Rf 0.5) zeigt vollständigen Umsatz an.

Die Lösung wird filtriert, eingeengt und das Produkt mit Hilfe der PSC gereinigt (Kieselgel Si 60, 1 mm, Laufmittel Dichlormethan/Aceton, 9:1, v/v; Rf 0.4).
Ausbeute: 48.0 mg = 71 % der Theorie.
$^1$H-NMR ($CDCl_3$): δ = 1.85 (m, 1 H, H-6); 2.45 (m, 1 H, H-8); 3.10 (q, 1 H, H-2); 3.11 (d, 1 H, H-4); 3.74 (s, 3-OH); 5.08 (d, 1 H, H-5);
1.46 (s, 9 H, BOC); 3.95 (d, 2 H, Glycin).
IR (Film): ν = 3539, 3402, 2975, 2935, 2829, 1750, 1723, 1519, 1461, 1370, 1230, 1168, 1093, 987, 701 $cm^{-1}$.
UV (Methanol: $\lambda_{max}$ (1g ε) = 207 nm (3.42)
MS (70 eV): m/e (%) = 677 (1, $M^+$), 659 (1), 645 (1), 502 (1), 470 (3), 414 (3), 296 (7), 157 (98), 91 (96), 57 (100).

| | | |
|---|---|---|
| Hochauflösung: | $C_{36}H_{55}NO_{11}$ | Ber.: 677.3775 |
| | | Gef.: 677.3788 $(M-1)^+$ |

b) Herstellung des Endprodukts

50 mg (75 µmol) des gemäss a) gewonnenen 5-Soraphenyl-(N-BOC)-glycinesters werden in 1 ml THF gelöst, mit 1 ml 6 N HCl versetzt und 2 h bei RT gerührt. Ein DC (Laufmittel Dichlormethan/Methanol, 9:1, v/v; Edukt Rf 0.8, Produkt Rf 0.4) zeigt eine vollständige Reaktion an. Die Lösung wird mit Natriumhydrogencarbonatlösung bis pH 7 neutralisiert, fünfmal mit Ethylacetat extrahiert und der Extrakt eingeengt. Das Rohprodukt wird mit Hilfe der PSC gereinigt (Kieselgel Si 60, 1 mm, Laufmittel Dichlormethan/Methanol, 9:1, v/v; Rf 0.2).
Ausbeute: 35.0 mg = 76 % (als Hydrochlorid)
$^1$H-NMR ($CDCl_3$): δ = 1.83 (m, 1 H, H-6); 2.44 (m, 1 H, H-8); 3.10 (q, 1 H, H-2); 3.11 (d, 1 H, H-4); 5.06 (d, 1 H, H-5); 3.26 (s, 2 H, Glycin).

$^{13}C$-NMR ($CDCl_3$): δ = 32.52 d, 35.25 d (C-6, C-8); 98.43 s (C-3); 170.97 s (C-1); 44.11 t, 173.34 s (Glycin).
IR (Film): ν = 3368, 2931, 2861, 2827, 1727, 1708, 1481, 1380, 1253, 1189, 1152, 1100, 1069, 992, 975, 738, 699 $cm^{-1}$.
UV (Methanol): $\lambda_{max}$ (1 g ε) = 205 nm (4.15)
MS (70 eV): m/e (%) = 577 [1(M-H)$^+$], 599 (1), 545 (5), 527 (3), 513 (2), 502 (1), 484 (1), 470 (3), 290 (10), 232 (9), 189 (34), 157 (100).

H-14. Herstellung des 5-O-Phenylthionocarbonats von Soraphen A (Verb. Nr. 173)

52 mg (0.1 mmol) Soraphen A und 32 mg Pyridin (4 eq) werden in 1 ml trockenem Dichlormethan gelöst und mit 35 mg Chlorthioameisensäure-O-phenylester (2 eq) versetzt. Nach 4 Tagen zeigt ein DC (Dichlormethan/Ether, 4:1, v/v; Edukt Rf 0.25, Produkt Rf 0.65) eine nahezu vollständige Umsetzung.

Die Lösung wird eingeengt und das Produkt mit Hilfe der PSC gereinigt (Kieselgel Si 60, 1 mm, Laufmittel Dichlormethan/Ether, 4:1, v/v; Rf 0.5).
Ausbeute: 45.7 mg = 70 %
$^1H$-NMR ($CDCl_3$): δ = 2.08 (m, 1 H, H-6); 2.48 (m, 1 H, H-8); 3.17 (q, 1 H, H-2); 3.37 (d, 1 H, H-4); 3.84 (s, 3-OH); 5.56 (d, 1 H, H-5); 7.16, 7.27, 7.42 (Phenylthionocarbonat).
$^{13}C$-NMR ($CDCl_3$): δ = 32.26 d, 35.26 d (C-6, C-8); 98.27 s (C-3); 170.98 s (C-1); 122.05 d, 126.67 d, 129.50 d, 153,36 s (Phenylthionocarbonat).
IR (Film): ν = 3537, 2971, 2935, 2863, 2829, 1752, 1594, 1461, 1332, 1282, 1226, 1091, 1062, 988, 971, 857, 699 $cm^{-1}$.
UV (Methanol): $\lambda_{max}$ (1 g ε) = 237 nm (3.84)
MS (70 eV): m/e (%) = 656 (1, M$^+$), 6.38 (1), 502 (4), 484 (2), 313 (4), 295 (13), 189 (32), 157 (96), 91 (99), 71 (100).

| | |
|---|---|
| Hochauflösung: $C_{36}H_{48}O_9S$ | Ber.: 656.3019 |
| | Gef.: 656.3034 M$^+$ |

H-15. Herstellung von 5-Desoxy-Soraphen A (Verb. Nr. 57)

38 mg (58 μmol) des gemäss Beispiel H-14 gewonnenen 5-O-Phenylthionocarbonats und 4 mg Azoisobutyronitril (0.1 eq) werden in 2 ml trockenem Toluol gelöst und mit 34 mg (2 eq) Tributylzinnhydrid versetzt. Die Lösung wird mit $N_2$ gespült und 1 h bei 80°C gerührt. Ein DC (Laufmittel Dichlormethan/Ether, 4:1, v/v; Edukt Rf 0.65, Produkt Rf 0.55) zeigt eine vollständige Umsetzung an.

Die Lösung wird eingeengt, der Rückstand in Dichlormethan aufgenommen, mit Natriumhydrogencarbonatlösung und 1 N HCl gewaschen und das Produkt mit Hilfe der PSC gereinigt (Kieselgel Si 60, 0.5 mm, Laufmittel Toluol/Methanol, 9:1, v/v; Rf 0.6).
Ausbeute: 15 mg = 51 %
$^1H$-NMR ($CDCl_3$): δ = 1.87 (M, 1 H, H-6); 1.91 (M, 1 H, H-5a); 1.98 (m, 1 H, H-5b); 2.42 (m, 1 H, H-6); 3.15 (d, 1 H, H-4); 3.19 (q, 1 H, H-2); 3.83 (s, 3-OH).
$^{13}C$-NMR ($CDCl_3$): δ = 26.73 t (C-5); 27.59 d (C-6); 35.78 d (C-8); 98.09 s (C-3); 171.45 s (C-1).
IR (Film): ν = 3540, 3035, 2937, 2892, 2825, 1752, 1662, 1602, 1462, 1362, 1243, 1218, 1150, 1122, 1095, 1058, 1008, 971, 906, 761, 736, 699 $cm^{-1}$.
UV (Methanol): $\lambda_{max}$ (1 g ε) = 202 nm (3.97)
MS (70 eV): m/e (%) = 504 (6, M$^+$), 472 (18), 297 (17), 269 (19), 267 (14), 217 (18), 189 (37), 157 (98), 71 (100).

| | |
|---|---|
| Hochauflösung: $C_{29}H_{44}O_7$ | Ber.: 504.3087 |
| | Gef.: 504.3090 M$^+$ |

H-16. Herstellung von (5R)-Soraphen A-β-D-glucopyranosid (Verb. Nr. 136)

a) Oxidation von Soraphen A zu Soraphen A-5-on

500 mg (0.84 mmol) Soraphen A-Etherat (M = 594.87) werden in 5 ml Dichlormethan gelöst und mit 300 mg (1.39 mmol) Pyridiniumchlorochromat versetzt. Man lässt 24 Stunden bei Raumtemperatur rühren und filtriert dann über Kieselgel 60 mit $CH_2Cl_2$/Aceton 95:5. Man erhält 375 mg (0.72 mmol, 86 %) des Produktes als blassgrünes Oel. Zur Charakterisierung kann das Rohprodukt mittels PSC (Merck, Kieselgel 60, Laufmittel: $CH_2Cl_2$/Aceton 95:5) gereinigt werden. $R_f$: 0.77 ($CH_2Cl_2$/Aceton = 90:10). M$^+$: 518
$^1H$-NMR ($CDCl_3$): δ = 3.23 (H-2); 3.18 (H-4); 2.56 (H-6); 4.01 (3-OH).

| $^{13}C$-NMR ($CDCl_3$): | Carbonyl-C | C-4/C-7/C-17 |
|---|---|---|
| | 207.2 | 74.8/75.7/82.4 (Zuordnung offen) |

b) Hydrierung von 5-Keto-Soraphen A zu (5R)-Soraphen A (= 5-epi-Soraphen A)

41 mg (0.073 mmol) Soraphen A-5-on werden in 2 ml abs. Methanol gelöst und mit 2.8 mg (0.073 mmol) Natriumborhydrid versetzt. Nach 1 stündigem Rühren bei Raumtemperatur wird die Reaktionsmischung mit 1 N HCl versetzt und mit Ethylacetat extrahiert. Die vereinigten organischen Phasen werden mit 5 %iger Natriumhydrogencarbonat- und konz. NaCl-Lösung gewaschen, über Natriumsulfat getrocknet und im Vakuum eingedampft. Man erhält 42 mg des Rohproduktes, das mittels PSC (Kieselgel 60, Lm: $CH_2Cl_2$/Aceton 90:10) gereinigt wird.

Man erhält 32 mg (0.06 mmol, 82 %) (5R)-Soraphen A als weissen Feststoff.

$R_f$: 0.44 ($CH_2Cl_2$/Aceton = 90:10)

$M^+$ = 520

$^1$H-NMR ($CDCl_3$): δ = 3.14 (H-2); 3.35 (H-4); 4.12 (H-5); 2.02 (H-6); 2.50 (H-8); 3.76 (3-OH); 1.89 (5-OH).

$^{13}$C-NMR ($CDCl_3$): Carbonyl-C C-4/C-5/C-7/C-17

170.9 70.3/74.7/76.8/79.9 (Zuordnung offen)

Diese zu Soraphen A epimere Verbindung gehört zum Erfindungsgegenstand der EP-A-282 455 und paralleler Patentanmeldungen.

c) Herstellung von (5R)-Soraphen A-2′,3′,4′,6′-tetra-O-acetyl-β-D-glucopyranosid

23 mg (44 μmol) des gemäss Vorschrift b) gewonnenen (5R)-Soraphens A, 100 mg gekörntes wasserfreies $CaSO_4$, 50 mg Silbersilikat (frisch hergestellt aus einer 1:1-Fällung von Na-Wasserglas und $AgNO_3$ und abgeschieden auf $Al_2O_3$) werden 30 min in 1 ml trockenem Dichlormethan gerührt. Dazu wird eine Lösung von 45 mg α-Acetobromglucose (2.5 eq) in 0.3 ml Dichlormethan gegeben und das Ganze 48 h bei RT gerührt. Ein DC (Laufmittel Dichlormethan/Aceton, 9:1, v/v; Edukt Rf 0.3, Produkt Rf 0.45) zeigt eine teilweise Umsetzung an.

Die Lösung wird filtriert, eingeengt und das Produkt mit Hilfe der PSC gereinigt (Kieselgel Si 60, 1 mm, Laufmittel Dichlormethan/Aceton, 9:1, v/v; Rf. 0.4).

Ausbeute: 4.5 mg = 12 %

$^1$H-NMR ($CDCl_3$): δ = 2.09 (m, 1 H, H-6); 2.53 (m, 1 H, H-8); 3.18 (q, 1 H, H-2); 3.33 (d, 1 H, H-4); 4.18 (m, 1 H, H-5); 3.92 (s, 3-OH);

2.00, 2.01, 2.05, 2.10 (s, 3 H, Tetraacetyl); 3.71, 4.16, 4.33, 4.60, 5.07, 5.12, 5.23 (Glucose).

MS (70 eV): m/e (%) = 850 (2, $M^+$), 832 (1), 818 (2), 470 (2), 331 (31), 217 (5), 189 (11), 169 (77), 157 (50), 43 (100).

d) Verseifung zum (5R)-Soraphen A-β-D-glucopyranosid

8.5 mg (10 μmol) (5R)-Soraphenyl-2′,3′,4′,6′-tetra-O-acetyl-β-D-glucopyranosid werden in 0.5 ml Methanol gelöst und mit 0.1 ml konz. Ammoniaklösung versetzt. Nach 2 h zeigt ein DC (Laufmittel Dichlormethan/Methanol, 9:1, v/v; Edukt Rf 0.8, Produkt Rf 0.2) eine vollständige Umsetzung an.

Das Produkt wird aus der Reaktionslösung direkt mit Hilfe der PSC gereinigt (Kieselgel Si 60, 0.5 mm, Laufmittel Dichlormethan/Methanol, 9:1, v/v; Rf 0.2).

Ausbeute: 2.2 mg = 33 %

$^1$H-NMR ($CDCl_3$): δ = 2.52 (m, 1 H, H-8); 3.19 (q, 1 H, H-2); 4.23 (m, 1H, H-5); 4.45 (d, 1 H, H-1″, Glucose)

MS (FAB): m/e (%) = 681 [30, (m-H)$^-$], 519 (7), 505 (20), 501 (15).

Analog werden das entsprechende β-D-Glukopyranosid und andere Saccharide des natürlichen Soraphens A gewonnen.

H-17. Herstellung von 5-tert. Butyldimethylsilyloxy-9,10-dihydro Soraphen A (Verb. Nr. 119)

100 mg der Verbindung Nr. 73 (s. Beipiel H-6) werden in 5 ml Methylenchlorid gelöst und unter Zusatz von 50 mg [Iridium (cyclooctadien) (acetonitril)(tricyclohexylphosphin)tetrafluoroborat während 1 Std bei 25° C und 1 atm $H_2$-Druck hydriert. Das Gemisch wird zwischen Ethylacetat und Wasser verteilt, getrocknet und eingeengt. Chromatographie an Kieselgel mit Ethylacetat/Hexan (1:4) als Laufmittel ergibt 76 mg (76 % der Theorie) der Verbindung Nr. 119.

MS: m/e = 636

$^1$H-NMR (250 MHz; $d_6$-Aceton):

5,86 (H-17); 5,22 (OH); 4,37 (OH); 3,98 (H-7).

$R_f$ (Ethylacetat/Hexan 1:3) = 0.19

H-18. Herstellung von 9,10-Dihydro-Soraphen A-5-formiat (Verb. Nr. 18)

a) Herstellung von 9,10-Dihydro-Soraphen A

35 mg der Verbindung Nr. 119 werden in 2 ml einer 1 · N Tetrabutylammoniumfluorid-Lösung in Tetrahydrofuran während 10 Minuten bei Raumtemperatur gerührt. Das Gemisch wird zwischen Ethylacetat und Wasser verteilt getrocknet und eingeengt. Chromatographie an Kieselgel mit Ethylacetat/Hexan (1:3) als Laufmittel liefert 22 mg Produkt.

$R_f$ (Ethylacetat/Hexan = 1:1) = 0,36

MS: m/e = 522

$^1$H-NMR (250 MH$_2$; $d_6$-Aceton):

5,84 (H-17); 5,36 (OH); 4,24 (OH); 3,98 (H-7).

b) Herstellung von 9,10-Dihydro-Soraphen A-5-formiat

25 mg der Verbindung Nr. 127 und 5.9 mg 4-Dimethylaminopyridin werden in 2 ml trockenem Dichlormethan gelöst und mit 5.9 mg HCOOH und 24.5 mg Triethylamin versetzt. Das Gemisch wird auf 10° C gekühlt, mit 10 mg Acetanhydrid versetzt und 30 min gerührt.

Das Eisbad wird entfernt und man lässt noch 30 min. bei Raumtemperatur rühren. Dann wird das Reaktionsgemisch mit einigen Tropfen Methanol versetzt und eingeengt. Der Rückstand wird mit Wasser versetzt und mehrmals mit Ethylacetat extrahiert. Die vereinigten organischen Phasen werden nacheinander mit 1N-HCl (pH 0), 5 %iger Natriumhydrogencarbonat-lösung (pH 8) und mit konz. Kochsalzlösung (pH 6) gewaschen. Dann wird über Natriumsulfat getrocknet und am Rotationsverdampfer eingeengt.

Man erhält 24.8 mg NMR-spektroskopisch einheitliches 9,10-Dihydro-Soraphen A-5-formiat, $R_f$ (Dichlormethan/Aceton = 9:1) 0.68.

Auf diese Art oder nach einer der weiter oben angegebenen Methoden lassen sich folgende Soraphen-Derivate der Formel I gewinnen. In der folgenden Tabelle kennzeichnet "DB" eine Doppelbindung in der 9.10-Position.

Tabelle 3

| Nr. | R | 9,10-Stellung | $R_o$ |
|---|---|---|---|
| 1 | $CH_3$ | DB | O–CHO |
| 2 | H | – | O–CHO |
| 3 | CHO | – | O–CHO |
| 4 | $CH_3$ | DB | O–CO–$CH_3$ |
| 5 | $CH_3$ | DB | O–CO–$CF_3$ |
| 6 | $CH_3$ | DB | O–CO–$C_2H_5$ |
| 7 | $CH_3$ | DB | O–CO–$C_3H_7$ |
| 8 | $CH_3$ | DB | O–CO–iso–$C_3H_7$ |
| 9 | $CH_3$ | DB | O–CO–$C_6H_{13}$ |
| 10 | $COCH_3$ | – | O–CO–$CH_3$ |
| 11 | $COC_3H_7$ | – | O–CO–$C_3H_7$ |
| 12 | H | – | O–CO–$CH_3$ |
| 13 | H | – | O–CO–$C_3H_7$ |
| 14 | H | – | O–CO–Phenyl |
| 15 | $CH_3$ | DB | O–CO–Phenyl |
| 16 | $COCF_3$ | – | O–CO–$CF_3$ |
| 17 | CO–Cyclohexyl | – | O–CO–Cyclohexyl |
| 18 | $CH_3$ | – | O–CHO |
| 19 | $CH_3$ | DB | O–CO–Benzyl |
| 20 | $CH_3$ | DB | O–CO–$CH_2$– 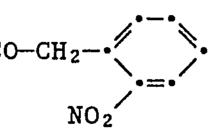 |
| 21 | H | – | O–CO–$CH_2$– 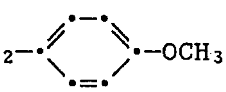 |
| 22 | $CH_3$ | DB | O–CO– 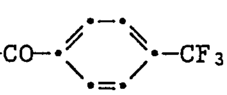 |
| 23 | $CH_3$ | DB | O–CO– 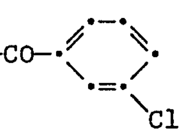 |
| 24 | $CH_3$ | DB | O–CO– 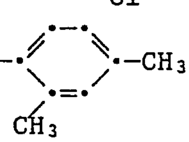 |

Tabelle 3 (Fortsetzung)

| Nr. | R | 9,10-Stellung | $R_o$ |
|---|---|---|---|
| 25 | $CH_3$ | DB | $O-CO-CCl_3$ |
| 26 | $CH_3$ | DB | $O-CO-CH_2SCH_3$ |
| 27 | $CH_3$ | DB | $O-CO-CH_2O$-Phenyl |
| 28 | $CH_3$ | DB | $O-CO-CH_2O$-Cyclopropyl |
| 29 | $CH_3$ | - | $O-CO-(CH_2)_6OC_3H_7$ |
| 30 | H | - | $O-CO-CH_2-C{\equiv}C-H$ |
| 31 | $CH_3$ | DB | $O-CO-(CH_2)_4-C{\equiv}C-H$ |
| 32 | $CH_3$ | DB | $O-CO-CH{=}CH_2$ |
| 33 | $COCH_3$ | - | $O-CO-(CH_2)_4-CH{=}CH_2$ |
| 34 | $COCF_3$ | - | $O-CO-CH{=}CH-CH_3$ (trans) |
| 35 | $CH_3$ | DB | $O-CS-CH_3$ |
| 36 | $CH_3$ | DB | O-CHS |
| 37 | $CH_3$ | DB | O-CS-Benzyl |
| 38 | $COCH_3$ | - | $O-CS-CH_2OCH_3$ |
| 39 | $CH_3$ | DB | O-CS-Cyclopropyl |
| 40 | H | - | O-CS-Cyclohexyl |
| 41 | $CH_3$ | DB | $O-CS-CH{=}CH_2$ |
| 42 | $CH_3$ | DB | $O-CS-C{\equiv}C-H$ |
| 43 | $COCH_3$ | - | $O-CO-CHCl_2$ |
| 44 | $CH_3$ | DB | $O-CO-CHF_2$ |
| 45 | H | - | $O-CO-CF_2Cl$ |
| 46 | $CH_3$ | DB | $O-CO-C_3F_7$ |
| 47 | $CH_3$ | DB | $O-CO-CH_2OCH_3$ |
| 48 | $CH_3$ | DB | $O-CO-C{\equiv}C-H$ |
| 49 | $CH_3$ | DB | $O-CO-CH(CH_3){=}CH(CH_3)$[Tiglat] |
| 50 | $COCH_2OCH_3$ | - | $O-CO-CH_2OCH_3$ |
| 51 | $CH_3$ | DB | O-CO-Cyclopropyl |
| 52 | $COCHCl_2$ | - | $O-CO-CHCl_2$ |
| 53 | $CH_3$ | DB | $O-CO-CH_2NH_2$ |
| 54 | $CH_3$ | DB | $O-CO-CH(CH_3)OH$ |
| 55 | $CH_3$ | DB | $O-CO-CH_2CH_2COOH$ |
| 56 | H | - | H |
| 57 | $CH_3$ | DB | H |

Tabelle 3 (Fortsetzung)

| Nr. | R | 9,10-Stellung | $R_o$ |
|---|---|---|---|
| 58 | $CH_3$ | - | H |
| 59 | $COCH_3$ | - | H |
| 60 | $CH_3$ | DB | F |
| 61 | $CH_3$ | DB | Cl |
| 62 | $CH_3$ | DB | Br |
| 63 | $CH_3$ | DB | I |
| 64 | $COCH_3$ | - | Cl |
| 65 | CO-Cyclopropyl | - | F |
| 66 | $CO(CH_2)_6I$ | - | H |
| 67 | $CH_3$ | DB | $O-Si(CH_3)_3$ |
| 68 | $CH_3$ | DB | $O-Si(CH_3)_2$(Phenyl) |
| 69 | H | - | $O-Si(CH_3)_2$(Thexyl) |
| 70 | CHO | - | $O-Si(Phenyl)_3$ |
| 71 | COCyclopropyl | - | $O-Si(CH_3)_2$-Cyclohexyl |
| 72 | $CH_3$ | DB | $O-Si(CH_3)_2$-Cyclohexyl |
| 73 | $CH_3$ | DB | $O-Si(CH_3)_2$-tert.Butyl |
| 74 | $CH_3$ | DB | $O-SO_2CH_3$ |
| 75 | $CH_3$ | DB | $O-SO_2-$ 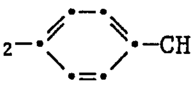 $-CH_3$ |
| 76 | H | - | $O-SO_2-C_4H_9$ |
| 77 | $CH_3$ | DB | $O-SO_2-$ 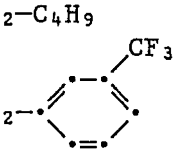 |
| 78 | $COCH_3$ | - | $O-SO_2-$ 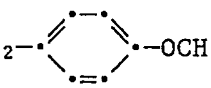 $-OCH_3$ |
| 79 | $CH_3$ | DB | $O-SO_2-$ 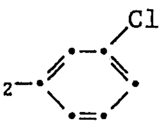 |
| 80 | $COCF_3$ | - | $O-SO_2$-Cyclohexyl |
| 81 | $CH_3$ | DB | $O-SO_3Na$ |
| 82 | $CH_3$ | DB | $O-CH_2-C\equiv C-H$ |
| 83 | $CH_3$ | - | $O-(CH_2)_4-C\equiv C-H$ |
| 84 | $CH_3$ | DB | $O-CH_2-CH=CH_2$ |
| 85 | $COCH_3$ | - | $O-(CH_2)_4-CH=CH_2$ |

Tabelle 3 (Fortsetzung)

| Nr. | R | 9,10-Stellung | $R_o$ |
|---|---|---|---|
| 86 | $COCH_2OCH_3$ | – | $O-CH_2-CH=CH_2$ |
| 87 | CHO | – | $O-CH_2-CH=CH-CH_3$ (trans) |
| 88 | $CH_3$ | DB | $O-CH_2-COOH$ |
| 89 | $CH_3$ | DB | $O-CH_2CH_2-NH_2$ |
| 90 | $CH_3$ | DB | $O-CH_2-CH(OH)$(Phenyl) |
| 91 | $CH_3$ | DB | $O-CH_2-CO-$Phenyl |
| 92 | $CH_3$ | DB | $O-CH_3$ |
| 93 | $CH_3$ | DB | $O-CH_2OCH_3$ |
| 94 | $CH_3$ | DB | $O-(CH_2)_6OC_3H_7n$ |
| 95 | $CH_3$ | DB | O–Cyclopropyl |
| 96 | $CH_3$ | DB | O–Cyclohexyl |
| 97 | $COCH_3$ | – | $O-CH_2-S-CH_3$ |
| 98 | H | – | $O-C_6H_{13}n$ |
| 99 | $CH_3$ | DB | O–Phenyl |
| 100 | $CH_3$ | DB | O–Benzyl |
| 101 | $CH_3$ | DB | $O-CH_2-$ 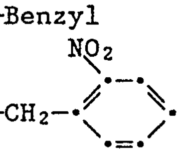 |
| 102 | H | – | $O-CH_2-$ 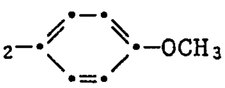 |
| 103 | $COCH_3$ | – | O– 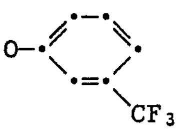 |
| 104 | $CH_3$ | DB | O– 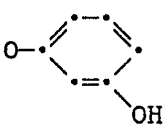 |
| 105 | $COCF_3$ | – | $-O-CH_2-$ 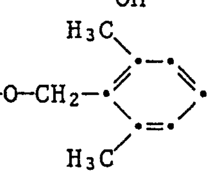 |
| 106 | $CH_3$ | DB | $O-CH_2-$ 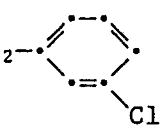 |
| 107 | $CH_3$ | DB | $OCH_2CH_2Br$ |

<u>Tabelle 3</u> (Fortsetzung)

| Nr. | R | 9,10-Stellung | $R_o$ |
|---|---|---|---|
| 108 | $CH_3$ | DB | $OCH_2CF_3$ |
| 109 | $CH_3$ | DB | $OCH_2-S-CH_3$ |
| 110 | $CH_3$ | - | $OCOCH_3$ |
| 111 | $CH_3$ | - | $OCOCF_3$ |
| 112 | $CH_3$ | - | $OCOCCl_3$ |
| 113 | $CH_3$ | - | $OCOCH_2OCH_3$ |
| 114 | $CH_3$ | - | $OCOC_6H_5$ |
| 115 | $CH_3$ | - | $OCS-C_6H_5$ |
| 116 | $CH_3$ | - | Br |
| 117 | $CH_3$ | DB | $N_3$ |
| 118 | H | - | $N_3$ |
| 119 | $CH_3$ | - | $OSi(CH_3)_2$ tert.Butyl |
| 120 | $CH_3$ | - | $OSO_2CH_3$ |
| 121 | $CH_3$ | - | $OCH_3$ |
| 122 | $CH_3$ | - | $OCH_2-$ 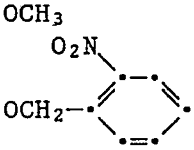 |
| 123 | $CH_3$ | - | $OCONH_2$ |
| 124 | $CH_3$ | - | 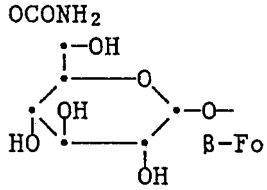 β-Form |
| 125 | $CH_3$ | DB | 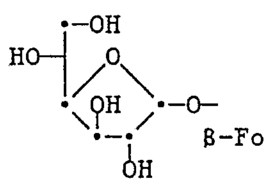 β-Form |
| 126 | $CH_3$ | - | $OCS-OCH_3$ |
| 127 | CHO | - | OH |
| 128 | $CH_3$ | DB | $O-C_6H_{13}$ |

Tabelle 3 (Fortsetzung)

| Nr. | R | 9,10-Stellung | $R_o$ |
|---|---|---|---|
| 129 | $CH_3$ | DB | 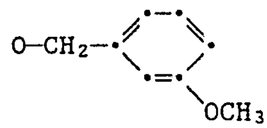 |
| 130 | $CH_3$ | DB | 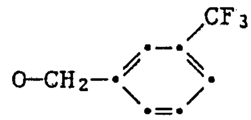 |
| 131 | $CH_3$ | DB | 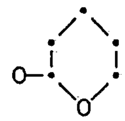 |
| 132 | $CH_3$ | DB | 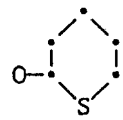 |
| 133 | H | – | 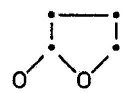 |
| 134 | $CH_3$ | DB | 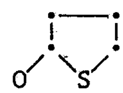 |
| 135 | $CH_3$ | DB | $O-CH_2OCH_2CH_2OCH_3$ |
| 136 | $CH_3$ | DB | 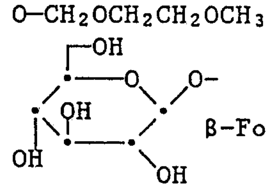 β-Form |
| 137 | $CH_3$ | DB | 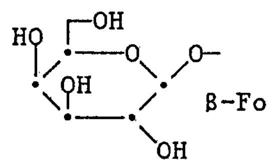 β-Form |
| 138 | $CH_3$ | DB | 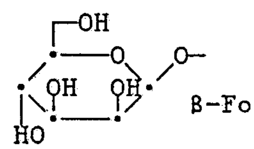 β-Form |
| 139 | $CH_3$ | DB | $O-CO-NH_2$ |
| 140 | H | – | $O-CO-NHCH_3$ |
| 141 | $CH_3$ | DB | O-CO-NH(Phenyl) |
| 142 | $CH_3$ | DB | O-CO-N(iso-Propyl) (Cyclohexyl) |
| 143 | $CH_3$ | DB | $O-CO-N(C_6H_{13}n)_2$ |
| 144 | $COCH_3$ | – | O-CO-NH(Cyclopro-pyl) |
| 145 | $CH_3$ | DB | $O-CS-NH_2$ |

Tabelle 3 (Fortsetzung)

| Nr. | R | 9,10-Stellung | $R_o$ |
|---|---|---|---|
| 146 | $CH_3$ | DB | O–CS–NH(Phenyl) |
| 147 | $CH_3$ | DB | O–CO–NH(Cyclopropyl) |
| 148 | $CH_3$ | DB | O–CO–$NHCH_3$ |
| 149 | $CH_3$ | DB | O–CO–$OCH_3$ |
| 150 | H | – | O–CO–$OC_6H_{13}$ |
| 151 | $CH_3$ | DB | O–CO–O–Cyclohexyl |
| 152 | $COCH_3$ | – | O–CO–O–Cyclopropyl |
| 153 | $CH_3$ | DB | O–CO–O–Phenyl |
| 154 | $CH_3$ | DB | O–CS–$OCH_3$ |
| 155 | H | – | O–CS–O–Phenyl |
| 156 | $CH_3$ | DB | O–CS–O–Cyclohexyl |
| 157 | $CH_3$ | DB | O–CO–$SCH_3$ |
| 158 | $CH_3$ | DB | O–CS–$SC_6H_{13}n$ |
| 159 | H | – | O–CO–S–Phenyl |
| 160 | $COCH_3$ | – | O–CS–S–Cyclohexyl |
| 161 | $CH_3$ | DB | O–CO–S–Cyclopropyl |
| 162 | $CH_3$ | DB | O–CS–S–iso-Propyl |
| 163 | $CH_3$ | DB | O–CO–S–Phenyl |
| 164 | $CH_3$ | DB | O–CS–$SCH_3$ |
| 165 | $COCH_3$ | – | OH |
| 166 | $COC_4H_9$ | – | OH |
| 167 | $COCH_3$ | – | OH |
| 168 | $COCH_2OCH_3$ | – | OH |
| 169 | CO-Cyclopropyl | – | OH |
| 170 | $CH_3$ | DB | O-CO-O-tert. $C_4H_9$ |
| 171 | $CH_3$ | DB | O-CO-$C(CH_3)_3$ |
| 172 | $CH_3$ | DB | O-2,4-Dinitrophenyl |
| 173 | $CH_3$ | DB | O-CS-O-Phenyl |
| 174 | $CH_3$ | DB | O-Si(OH)(tert.butyl)$_2$ |
| 175 | $CH_3$ | – | O-CO-$CCl_3$ |
| 176 | H | – | O-CO-$CCl_3$ |
| 177 | $CH_3$ | – | O-CO-$CH_2NH_2$ |
| 178 | H | – | O-CO-$CH_2NH_2$ |

2. Formulierungsbeispiele für den Wirkstoff der Formel I (% = Gewichtsprozent) ["Wirkstoff" bedeutet im folgenden einen Wirkstoff aus vorstehender Tabelle]

| 2.1 Emulsions-Konzentrate | a) | b) | c) |
|---|---|---|---|
| Wirkstoff | 25 % | 40 % | 50 % |
| Ca-Dodecylbenzolsulfonat | 5 % | 8 % | 6 % |
| Ricinusöl-polyethylenglykolether (36 Mol Ethylenoxid) | 5 % | - | - |
| Tributylphenol-polyethylenglykolether (30 Mol Ethylenoxid) | - | 12 % | 4 % |
| Cyclohexanon | - | 15 % | 20 % |
| Xylolgemisch | 65 % | 25 % | 20 % |

Aus solchen Konzentration können durch Verdünnen mit Wasser Emulsionen jeder gewünschten Konzentration hergestellt werden.

| 2.2 Lösungen | a) | b) | c) | d) |
|---|---|---|---|---|
| Wirkstoff | 80 % | 10 % | 5 % | 95 % |
| Ethylenglykol-monomethyl-ether | 20 % | - | - | - |
| Polyethylenglykol MG 400 | - | 70 % | - | - |
| N-Methyl-2-pyrrolidon | - | 20 % | - | - |
| Epoxydiertes Kokosnussöl | - | - | 1 % | 5 % |
| Benzin (Siedegrenzen 160-190° C) | - | - | 94 % | - |

(MG = Molekulargewicht)

Die Lösungen sind zur Anwendung in Form kleinster Tropfen geeignet.

| 2.3 Granulate | a) | b) |
|---|---|---|
| Wirkstoff | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

| 2.4 Stäubemittel | a) | b) |
|---|---|---|
| Wirkstoff | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel. Diese lassen sich durch weiteren Zusatz der drei Trägerstoffe auf anwendungsfertige Stäube mit 0,001 % Wirkstoff vermahlen.

2.5 Spritzpulver

| | a) | b) | c) |
|---|---|---|---|
| Wirkstoff | 25 % | 50 % | 75 % |
| Na-Ligninsulfonat | 5 % | 5 % | - % |
| Na-Laurylsulfat | 3 % | - | 5 % |
| Na-Diisobutylnaphthalinsulfonat | - | 6 % | 10 % |
| Octylphenolpolyethylenglykolether (7-8 Mol Ethylenoxid) | - | 2 % | - % |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

2.6 Umhüllungs-Granulat

| | |
|---|---|
| Wirkstoff | 3 % |
| Polyethylenglykol (MG 200) | 3 % |
| Kaolin | 94 % |

(MG = Molekulargewicht)

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

2.7 Suspensions-Konzentrat

| | |
|---|---|
| Wirkstoff | 40 % |
| Ethylenglykol | 10 % |
| Nonylphenolpolyethylenglykolether (15 Mol Ethylenoxid) | 6 % |
| Na-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

3. Biologische Beispiele an Pflanzen
(Im folgenden bedeutet "Wirkstoff" ohne weiteren Hinweis ein Präparat aus der Tabelle 3.)

Beispiele 3.1: Wirkung gegen Puccinia graminis auf Weizen

a) Residual-protektive Wirkung
Weizenpflanzen werden 6 Tage nach der Aussaat mit einer aus Spritzpulver des Wirkstoffes hergestellten Spritzbrühe (0,02 % Aktivsubstanz) besprüht. Nach 24 Stunden werden die behandelten Pflanzen mit einer Uredosporensuspension des Pilzes infiziert. Nach einer Inkubation während 48 Stunden bei 95-100 % relativer Luftfeuchtigkeit und ca. 20° C werden die infizierten Pflanzen in einem Gewächshaus bei ca. 22° C aufgestellt. Die Beurteilung der Rostpustelnentwicklung erfolgt 12 Tage nach der Infektion.

b) Systemische Wirkung
Zu Weizenpflanzen wird 5 Tage nach der Aussaat eine aus Spritzpulver des Wirkstoffes hergestellte Spritzbrühe gegossen (0,002 % Aktivsubstanz bezogen auf das Bodenvolumen). Nach 48 Stunden werden die behandelten Pflanzen mit einer Uredosporensuspension des Pilzes infiziert. Nach einer Inkubation während 48 Stunden bei 95-100 % relativer Luftfeuchtigkeit und ca. 20°C werden die infizierten Pflanzen in einem

Gewächshaus bei ca. 22°C aufgestellt. Die Beurteilig der Rostpustelnentwicklung erfolgt 12 Tage nach der Infektion.

Der Pilzbefall wurde in beiden Versuchen vollständig durch Wirkstoffe der Formel I gehemmt. Im Versuch a) zeigten die Verbindungen Nr. 2, 18, 25, 47, 55, 175, 176, 113, 136, 177, 178 und andere auch noch in einer Verdünnung von 0,006 % vollständige Hemmung (0 - 5 % Pilzbefall).

Unbehandelte aber infizierte Kontrollpflanzen wiesen dagegen einen Puccinia-Befall von 100 % auf.

Beispiel 3.2: Wirkung gegen Phytophthora auf Tomatenpflanzen

a) Residual-protektive Wirkung

Tomatenpflanzen wurden nach 3-wöchiger Anzucht mit einer aus Spritzpulver des Wirkstoffes hergestellten Spritzbrühe (0,02 % Aktivsubstanz) besprüht. Nach 24 Stunden wurden die behandelten Pflanzen mit einer Sporangiensuspension des Pilzes infiziert. Die Beurteilung des Pilzbefalls erfolgte nach einer Inkubation der infizierten Pflanzen während 5 Tagen bei 90-100 % relativer Luftfeuchtigkeit und 20°C.

b) Systemische Wirkung

Zu Tomatenpflanzen wurde nach 3-wöchiger Anzucht eine aus Spritzpulver des Wirkstoffes hergestellte Spritzbrühe gegossen (0,002 % Aktivsubstanz bezogen auf das Erdvolumen). Es wurde dabei darauf geachtet, dass die Spritzbrühe nicht mit den oberirdischen Pflanzenteilen in Berührung kam. Nach 48 Stunden wurden die behandelten Pflanzen mit einer Sporangiensuspension des Pilzes infiziert. Die Beurteilung des Pilzbefalls erfolgte nach einer Inkubation der infizierten Pflanzen während 5 Tagen bei 90-100 % relativer Lufffeuchtigkeit und 20°C.

In beiden Versuchen wurde bei der Auswertung kein Pilzbefall beobachtet. Im Versuch a) bewirkten die Verbindungen Nr. 1, 2, 18, 25, 47, 55, 113, 136, 175, 176 und andere auch noch in einer Verdünnung von 0,006 % vollständige Hemmung des Krankheitsbefalls (= 0 - 5 % Befall).

Beispiel 3.3: Wirkung gegen Plasmopara viticola auf Reben

Residual-protektive Wirkung

Im 4-5 Blattstadium werden Rebensämlinge mit einer aus Spritzpulver des Wirkstoffes hergestellten Spritzbrühe (0,006 % Aktivsubstanz) besprüht. Nach 24 Stunden werden die behandelten Pflanzen mit einer Sporangiensuspension des Pilzes infiziert. Nach einer Inkubation während 6 Tagen bei 95-100 % relativer Luftfeuchtigkeit und 20°C wird der Pilzbefall beurteilt.

Im Gegensatz zu den unbehandelten, aber infizierten Kontrollpflanzen mit 100 % Pilzbefall waren die mit Wirkstoff I behandelten Pflanzen befallsfrei, z.B. die mit Verbindung Nr. 1, 2, 4, 10, 18, 25, 47, 113, 136, 175 oder 176 behandelten.

Beispiel 3.4: Wirkung gegen Cercospora arachidicola auf Erdnusspflanzen

Residual protektive Wirkung

10-15 cm hohe Erdnusspflanzen werden mit einer aus Spritzpulver der Wirksubstanz hergestellten Spritzbrühe (0,006 % Aktivsubstanz) besprüht und 48 Stunden später mit einer Konidiensuspension des Pilzes infiziert. Die infizierten Pflanzen werden während 72 Stunden bei ca. 21°C und hoher Luftfeuchtigkeit inkubiert und anschliessend bis zum Auftreten der typischen Blattflecken in einem Gewächshaus aufgestellt. Die Beurteilung der fungiziden Wirkung erfolgt 12 Tage nach der Infektion basierend auf Anzahl und Grösse der auftretenden Flecken.

Die mit dem Wirkstoff I, z.B. mit Verbindung Nr. 1, 2, 4, 8, 10, 18, 25, 53, 47, 55, 73, 113, 131, 136, 172, 175, 176, 177 und 178 behandelten Pflanzen waren ohne Befall. Unbehandelte aber infizierte Kontrollpflanzen wiesen dagegen einen Cercospora-Befall von 100 % auf.

Beispiel 3.5: Wirkung gegen Venturia inaequalis auf Apfeltrieben

Residual-protektive Wirkung

Apfelstecklinge mit 10-20 cm langen Frischtrieben werden mit einer aus Spritzpulver des Wirkstoffes hergestellten Spritzbrühe (0,02 % Aktivsubstanz) besprüht. Nach 24 Stunden werden die behandelten Pflanzen mit einer Konidiensuspension des Pilzes infiziert. Die Pflanzen werden dann während 5 Tagen bei 90-100 % relativer Luftfeuchtigkeit inkubiert und während 10 weiteren Tagen in einem Gewächshaus bei 20-24°C aufgestellt. Der Schorfbefall wird 15 Tage nach der Infektion beurteilt.

Die mit Wirkstoff I behandelten Stecklinge waren befallsfrei. Mit den Verbindungen Nr. 1, 2, 6, 7, 10, 18, 25, 47, 49, 55, 84, 113, 136, 177, 53 wurde auch noch in einer Verdünnung von 0,006 % der Krankheitsbefall vollständig verhindert (0 - 5 % Befall).

Beispiel 3.6: Wirkung gegen Botrytis cinerea an Apfelfrüchten

Residual-protektive Wirkung

Künstlich verletzte Aepfel werden behandelt, indem eine aus Spritzpulver der Wirksubstanz hergestellte Spritzbrühe (0,02 % Aktivsubstanz) auf die Verletzungsstellen aufgetropft wird. Die behandelten Früchte werden anschliessend mit einer Sporensuspension des Pilzes inokuliert und während einer Woche bei hoher Luftfeuchtigkeit und ca. 20°C inkubiert. Bei der Auswertung werden die angefaulten Verletzungsstellen gezählt und daraus die fungizide Wirkung der Testsubstanz abgeleitet.

Der Wirkstoff I hemmte den Pilzwuchs vollständig. Mit den Verbindungen Nr. 1, 2, 4, 6, 7, 8, 15, 18, 25, 32, 47, 49, 55, 57, 73, 74, 75, 84, 92, 53, 113, 124, 131, 136, 139, 141, 149, 165, 168 und 170 - 178 wurde auch noch in einer Verdünnung von 0,006 % der Krankheitsbefall vollständig verhindert (0 - 5 % Befall).

Beispiel 3.7: Wirkung gegen Erysiphae graminis auf Gerste

a) Residual-protektive Wirkung

Ca. 8 cm hohe Gerstenpflanzen werden mit einer aus Spritzpulver des Wirkstoffes hergestellten Spritzbrühe (0,006 % Aktivsubstanz) besprüht. Nach 3-4 Stunden werden die behandelten Pflanzen mit Konidien des Pilzes bestäubt. Die infizierte Gerstenpflanzen werden in einem Gewächshaus bei ca. 22° C aufgestellt und der Pilzbefall nach 10 Tagen beurteilt.

b) Systemische Wirkung

Zu ca. 8 cm hohen Gerstenpflanzen wird eine aus Spritzpulver des Wirkstoffes hergestellte Spritzbrühe gegossen (0,002 % Aktivsubstanz bezogen auf das Erdvolumen). Es wurde dabei darauf geachtet, dass die Spritzbrühe nicht mit den oberirdischen Pflanzenteilen in Berührung kam. Nach 48 Stunden werden die behandelten Pflanzen mit Konidien des Pilzes bestäubt. Die infizierten Gerstenpflanzen werden in einem Gewächshaus bei ca. 22°C aufgestellt und der Pilzbefall nach 10 Tagen beurteilt.

Die Pflanzen waren in beiden Versuchen befallsfrei, die Kontrollpflanzen vollständig befallen. Im Versuch a) bewirkten die Verbindungen Nr. 1, 3 - 8, 12 - 15, 18, 22, 25, 32, 47, 49, 50, 53, 55, 57, 67, 73, 74, 75, 84, 92, 100, 113, 124, 127, 131, 136, 139, 141, 149, 165, 168 und 170 - 178 auch noch in einer Verdünnung von 0,002 % eine vollständige Hemmung des Krankheitsbefalls (0 - 5 %).

Beispiel 3.8: Wirkung gegen Rhizoctonia solani (Bodenpilz auf Reispflanzen)

Protektiv-lokale Bodenapplikation

12 Tage alte Reispflanzen werden mit einer Formulierung des Wirkstoffes hergestellten Spritzbrühe (0,002 % Aktivsubstanz), ohne oberirdische Pflanzenteile zu kontaminieren, angegossen. Zur Infizierung der behandelten Pflanzen wird eine Suspension von Myzel und Sklerotien von R. solani auf die Bodenoberfläche gegeben. Nach 6-tägiger Inkubation bei 27°C (Tag), bzw. 23°C (Nacht) und 100 % rel. Luftfeuchtigkeit (Feuchtkasten) in der Klimakammer wird der Pilzbefall auf Blattscheide, Blättern und Stengel beurteilt.

Nach Behandlung mit dem Wirkstoff I trat kein Befall auf.

Beispiel 3.9: Wirkung gegen Pyricularia oryzae auf Reispflanzen

a) Residual-protektive Wirkung

Reispflanzen werden nach zweiwöchiger Anzucht mit einer aus Spritzpulver des Wirkstoffes hergestellten Spritzbrühe (0,006 % Aktivsubstanz) besprüht. Nach 48 Stunden werden die behandelten Pflanzen mit einer Konidiensuspension des Pilzes infiziert. Nach 5 Tagen Inkubation bei 95 - 100 % relativer Luftfeuchtigkeit und 24°C wird der Pilzbefall beurteilt.

b) Systemische Wirkung

Zu zwei Wochen alten in Blumentöpfen wachsenden Reispflanzen wird eine aus Spritzpulver des Wirkstoffes hergestellte Spritzbrühe gegossen (0,002 % Aktivsubstanz, bezogen auf das Erdvolumen). Darauf werden die Töpfe mit Wasser soweit gefüllt, dass die untersten Stengelteile der Reispflanzen im Wasser stehen. Nach 48 Stunden werden die behandelten Reispflanzen mit einer Konidiensuspension des Pilzes infiziert. Nach einer Inkubation der infizierten Pflanzen während 5 Tagen bei 95 - 100 % relativer Luftfeuchtigkeit und ca. 24° wird der Pilzbefall beurteilt.

Verbindungen aus den Tabellen zeigten nachhaltige Wirkung gegen den Pyricularia-Pilz (unter 20 % Befall). Unbehandelte aber infizierte Kontrollpflanzen wiesen dagegen einen Befall von 100 % auf.

Mit den Verbindungen Nr. 1, 2, 4, 6, 7, 8, 18, 25, 47, 55, 124 und anderen wurde vollständige Hemmung des Krankheitsbefalls erzielt (0 - 5 % Befall).

**Patentansprüche**

1. Macrocyclische Verbindung der Formel I

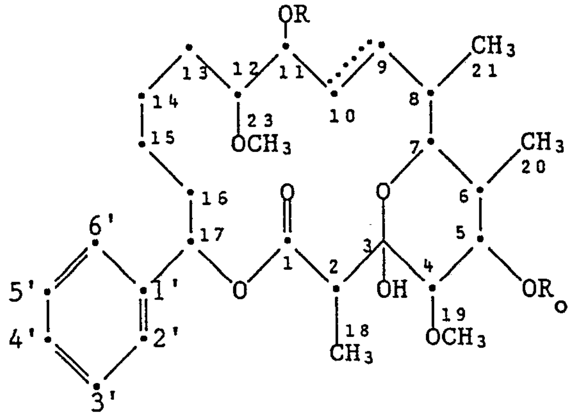

(I)

worin die gepunktete Linie in 9,10-Stellung wahlweise eine gesättigte Bindung oder eine Doppelbindung bedeutet, während

R Wasserstoff, $CH_3$ oder -COA ist, worin

A Wasserstoff, $C_3$-$C_6$Cycloalkyl, unsubstituiertes oder durch Halogen oder $C_1$-$C_3$Alkoxy substituiertes $C_1$-$C_6$-Alkyl darstellt,

$R_o$ Wasserstoff, Halogen, -$N_3$, -SH, -OH oder -OY bedeutet, und

Y wahlweise

a) eine $C_3$-$C_6$Alkenyl- oder Alkinylgruppe, eine unsubstituierte oder substituierte $C_1$-$C_6$Alkylgruppe, eine Alkoxyalkoxyalkylgruppe mit bis zu 10 C-Atomen, eine $C_3$-$C_6$Cycloalkylgruppe oder eine unsubstituierte oder substituierte Phenylgruppe bedeutet, oder

b) eine Silylgruppe -$SiR_1R_2R_3$ darstellt, in der $R_1$-$R_3$ unabhängig Alkyl, Phenyl, Benzyl oder $C_3$-$C_6$Cycloalkyl bedeuten, oder

c) -$SO_2Z$ darstellt, worin Z die Gruppe -OM bedeutet, in der M Wasserstoff oder das Moläquivalent eines Metalles ist, oder worin Z $C_1$-$C_6$Alkyl oder unsubstituiertes oder substituiertes Aryl bedeutet; oder

d) eine Gruppe

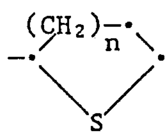

mit n = 1 oder 2 oder eine unsubstituierte oder nach Art einer Furanose oder Pyranose ganz oder teilweise durch -OH substituierte Gruppe

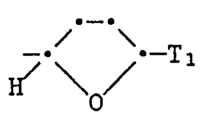

oder

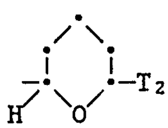

in der $T_1$ Wasserstoff, -OH, -$CH_2OH$ oder -CHOH-$CH_2OH$ und $T_2$ Wasserstoff, -OH oder -$CH_2OH$ sind,

darstellt, oder

e) eine (Thio)Acylgruppe -CO-B oder -CS-B bedeutet, worin

B Wasserstoff, unsubstituiertes oder substituiertes $C_1$-$C_6$Alkyl, oder aber $C_2$-$C_6$Alkenyl, $C_2$-$C_6$Alkinyl, $C_3$-$C_6$Cycloalkyl, unsubstituiertes oder substituiertes Phenyl oder eine der Gruppen -$N(R_4)(R_5)$, -$OR_6$ oder -$SR_6$ darstellt,

$R_4$ und $R_5$ unabhängig voneinander Wasserstoff, $C_1$-$C_6$Alkyl, $C_1$-$C_6$Haloalkyl, $C_3$-$C_6$Alkenyl, $C_3$-$C_6$Alkinyl, $C_3$-$C_6$Cycloalkyl oder Phenyl bedeuten, und

$R_6$ $C_1$-$C_6$Alkyl, $C_3$-$C_6$Cycloalkyl oder Phenyl ist, mit der Massgabe, dass R Methyl bedeutet, wenn in 9,10-Stellung eine Doppelbindung vorliegt, und dass R die Gruppe -COA darstellt, wenn $R_0$ eine OH-Gruppe ist.

2. Eine Verbindung gemäss Anspruch 1, in der R Methyl und $R_0$ Halogen, Azido, Thiol, Hydroxy oder OY bedeuten, worin Y die unter a), b), c), d) und e) genannten Bedeutungen besitzt.

3. Eine Verbindung gemäss Anspruch 2, worin Y die unter a) genannte Bedeutung besitzt.

4. Eine Verbindung gemäss Anspruch 2, worin Y die unter d) genannte Bedeutung besitzt.

5. Eine Verbindung gemäss Anspruch 4, worin Y einen durch Hydroxy und -$CH_2OH$ substituierten Tetrahydrofuranyl- oder Tetrahydropyranyl-Ring darstellt.

6. Soraphenyl-β-D-glucopyranosid und (5R)-Soraphenyl-β-D-glucopyranosid gemäss Anspruch 5.

7. Eine Verbindung gemäss Anspruch 2, worin Y die unter e) genannte Acylierung bedeutet.

8. Eine Verbindung gemäss Anspruch 7, ausgewählt aus Soraphen A-5-methoxyacetat, Soraphen A-5-trichloracetat, Soraphen A-5-formiat, Soraphen A-5-aminoacetat, 9,10-Dihydro-Soraphen A-5-formiat, 9,10-Dihydro-Soraphen A-5-methoxyacetat, 9,10-Dihydro-Soraphen A-5-aminoacetat.

9. Eine Verbindung gemäss Anspruch 1, in der
R Wasserstoff oder die Gruppe -COA darstellt, worin
A Wasserstoff, $C_3$-$C_6$Cycloalkyl, unsubstituiertes oder durch Halogen oder $C_1$-$C_3$Alkoxy substituiertes $C_1$-$C_6$Alkyl bedeutet, während
$R_0$ Wasserstoff, Azido, Halogen, Hydroxy oder Thiol ist.

10. Verfahren zur Herstellung einer Verbindung der Formel I, gekennzeichnet durch entsprechende Verätherung, Silylierung oder Acylierung der 5-Hydroxygruppe in einer Verbindung der Formel

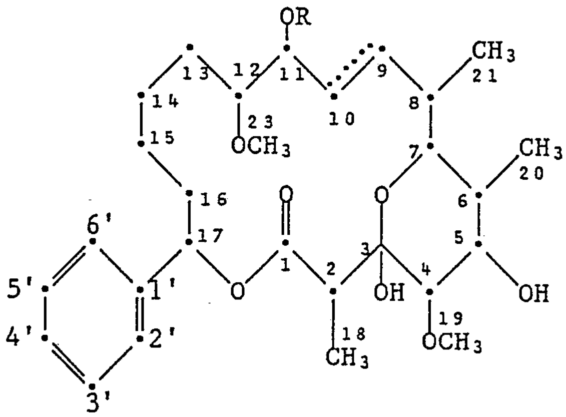

worin R Methyl bedeutet, wenn in 9,10-Stellung eine Doppelbindung steht, oder worin R Methyl oder Wasserstoff bedeutet, wenn in 9,10-Stellung eine Einfachbindung steht,
und/oder durch entsprechende Acylierung einer in 11-Stellung befindlichen Hydroxygruppe.

11. Mittel zur Bekämpfung oder Verhütung von Pflanzenkrankheiten, dadurch gekennzeichnet, dass es als mindestens einen Wirkstoff einer Verbindung der Formel I gemäss Anspruch 1 enthält, zusammen mit einem geeigneten Trägermaterial.

12. Mittel gemäss Anspruch 11 enthaltend einen Wirkstoff gemäss einem der Ansprüche 2 bis 9.

13. Verwendung von Verbindungen der Formel I gemäss Anspruch 1 zur Bekämpfung bzw. Verhütung von Pflanzenkrankheiten.

14. Verfahren zur Bekämpfung oder Verhütung von Pflanzenkrankheiten, dadurch gekennzeichnet, dass mindestens eine Verbindung der Formel I gemäss Anspruch 1 auf die Pflanze, auf Pflanzenteile oder ihren Standort appliziert wird.

15. Verfahren gemäss Anspruch 14, wobei die Pflanzenkrankheit durch Pilze verursacht wird.